# EUROPÄISCHE PATENTSCHRIFT

(11) **EP 1 228 199 B1**
(45) Veröffentlichungstag und Bekanntmachung des Hinweises auf die Patenterteilung: **19.04.2006**
(21) Anmeldenummer: 00971419.7
(22) Anmeldetag: 03.11.2000
(51) Int. Cl.: C12N 15/00, C12N 15/86

(54) **VERFAHREN ZUR GERICHTETEN VERPACKUNG VON MOLEKULAREN SUBSTANZEN IN PROTEINHÜLLEN**
METHOD FOR DIRECTED PACKAGING OF MOLECULAR SUBSTANCES IN PROTEIN SHELLS
PROCEDE D'ENCAPSULATION CIBLEE DE SUBSTANCES MOLECULAIRES DANS DES ENVELOPPES PROTEIQUES

(30) Priorität: 03.11.1999 DE 19952982
(43) Veröffentlichungstag der Anmeldung: 07.08.2002
(73) Patentinhaber: ACGT Progenomics AG, 06120 Halle (Saale) (DE)
(72) Erfinder: BÖHM, Gerald, 06114 Halle (Saale) (DE); ESSER, Dirk, Cherry Hinton, Cambridge CB1 9EW (GB); SCHMIDT, Ulrich, West Leederville, WA 6007 (AU)
(74) Vertreter: Behnisch, Werner
(86) Internationale Anmeldenummer: PCT/EP2000/010878
(87) Internationale Veröffentlichungsnummer: WO 2001/032852

(56) Entgegenhaltungen:
- WO-A-99/25324
- WEI-CHIH OU ET AL.: "The major capsid protein, VP1, of human JC virus expressed in Escherichia coli is able to self-assemble into a capsid-like particle and deliver exogenous DNA into human kidney cells" JOURNAL OF GENERAL VIROLOGY, Bd. 80, 1999, Seiten 39-46, XP002164832

## Beschreibung

Die vorliegende Erfindung betrifft ein Verfahren zur Verpackung von molekularen Substanzen in Proteinhüllen.

### Gebiet der Erfindung und Stand der Technik

Die *in vitro*-Verpackung von Nukleinsäuren oder anderen Wirkstoffen in Proteinhüllen besitzt hohe Bedeutung für biotechnologische Anwendungen, insbesondere für die Einbringung heterologer DNA in eukaryontische Zellen zum Studium der Zellbiologie, oder bei medizinischen Anwendungen für therapeutische Zwecke im Rahmen einer Gentherapie. Dabei werden zur *in vitro*-Verpackung oft einfache, rekombinant hergestellte Virushüllen herangezogen, da sie natürliche Nukleinsäureveipackungseinheiten darstellen; entsprechende Systeme werden als virusanaloge Gentransfersysteme bezeichnet. Sie zählen zu den physikalischen Gentransfersystemen, im Gegensatz zu den liposomalen Systemen (basierend auf verschiedenen Formulierungen von meist kationischen Lipiden und Detergentien, die zu Liposomen und verwandten Strukturen zusammengelagert werden) und zu den viralen Vektorsystemen. Die viralen Vektorsysteme für Gentransfer und Gentherapie sind heute zumeist von Retroviren abgeleitet, zunehmend werden aber auch andere Virustypen (Herpesvirus, Adenovirus, Adenoassoziiertes Virus) verwendet. Eine Zusammenfassung zu den verschiedenen Gentransfersysteme findet sich im Ersten TAB-Sachstandsbericht "Stand und Perspektiven naturwissenschaftlicher und medizinischer Problemlösungen bei der Entwicklung gentherapeutischer Heilmethoden" des Büros für Technikfolgenabschätzung beim Deutschen Bundestag (TAB-Arbeitsbericht Nr. 25, 1994).

Die natürliche Verpackung von Nukleinsäuren in Virushüllen ist ein komplexer Prozeß, bei dem viele Faktoren, unter anderem auch solche aus der Wirtszelle, miteinander interagieren. Der Prozeß ist in den meisten Fällen noch weitgehend unverstanden. Daher funktionieren die meisten darauf aufbauenden *in vitro*-Verpackungssysteme nur mit vergleichsweise geringe Effizienz und Ausbeute. Darüber hinaus wird bei den *in vitro*-Verfahren in der Regel ein Größenlimit bezüglich der zu verpackenden DNA beobachtet. Die geringe Effizienz der *in* vitro-Methoden im Vergleich zum natürlichen Verpackungsvorgang bei der Vinusentstehung ist meist auf das Fehlen von spezifischen Wirtsfaktoren im artifiziellen *in vitro*-System zurückzuführen.

Ein besonderes Hemmnis ist auch die Länge von unkondensierter DNA üblicher Größe in wäßriger Lösung. In diesem Zustand erreichen Plasmide (aufgrund der Abstoßung der negativen Ladungen des vergleichsweise steifen Polyphosphatrückgrates) bei einem Kodierungsumfang von 5 kbp eine lineare (hydrodynamische) Größe im Bereich von Mikrometern. Bei typischen ikosaedrischen Virushülldurchmessern von 30 bis 100 nm wird somit verständlich, daß ohne DNA-Kondensierung eine effiziente Verpackung kaum möglich ist. Andererseits führt der einfache Zusatz von Kondensationsmitteln zur Kapsid- oder Kapsomer-Lösung nicht zwingend zu verbesserten Verpackungseffizienzen. Solchermaßen kondensierte DNA neigt in typischen Konzentrationsbereichen zur Bildung von sogenannten *Toroiden,* das sind ringförmige, hochmolekulare Aggregate, die einen Durchmesser von mehreren Mikrometern besitzen und somit aufgrund ihrer Größe nicht in übliche virusanaloge Proteinhüllen verpackbar sind.

Schließlich existieren nur wenige beschriebene Verfahren zur Verpackung von Nukleinsäuren in virusanaloge Partikel *in vitro,* d. h. ohne die Verwendung spezieller Verpackungszellinien Ein diesbezüglich gut charakterisiertes System ist das von murinem Polyomavirus (vgl. S.N. Slilaty & H.V. Aposhian, "Gene transfer by polyoma-like particles assembled in a cell-free system", *Science* 220, S. 725-727, 1983; J. Forstova, N. Krauzewicz, V. Sandig, J. Elliott, Z. Palkova, M. Strauss & B.E. Griffin, "Polyoma virus pseudocapsids as efficient carriers of heterologous DNA into mammalian cells", *Hum. Gene Ther.* 6, S. 297-306, 1995). Das pentamere Hüllprotein VP 1 des Polyomavirus (PyVP1) kann unter geeigneten Lösungsmittelbedingungen (Erhöhung der Ionenstärke, Zusatz von Kalzium oder Ammoniumsulfat, Wahl eines geeigneten pH-Wertes) *in vitro* virusähnliche Strukturen ausbilden, die strukturell wegehend identisch zu den unter biologischen Bedingungen entstehenden Viruskapsiden sind. Eine Beladung der solchermaßen hergestellten Kapside kann durch eine seit langem beschriebene Prozedur des osmotischen Schocks ausgelöst werden (S.M. Barr, K. Keck & H.V. Aposhian, "Cell-free assembly of a polyoma-like panticle from empty capside and DNA", Vi*rology* 96, S. 656-659, 1979: S.N. Slilaty, K.I. Berns & H.V. Aposhian, "Polyoma-like particle: characterization of the DNA encapsidated *in vitro* by polyoma empty capsids", *J. Biol. Chem.* 257, S. 6571-6575, 1982). Dabei werden mit Kapsiden inkubierte Nukleinsäuremoleküle vor einem Nukleaseverdau geschützt. Vermutlich geschieht dies jedoch weniger durch eine Verpackung in das Innere des Kapsids, sondern vielmehr durch eine Auflagerung der Nukleinsäuren auf die Kapsidoberfläche. Dieses Verfahren des osmotischen Schocks ist darüber hinaus sehr ineffizient. Neuere Untersuchungen zeigen, daß im Gegensatz zu früheren Annahmen durch die Prozedur des osmotischen Schocks zwar einzelne Nukleinsäuren in Lösung statistisch in das Kapsid eingebaut werden können (H. Braun, K. Boller, J. Lower, W.M Bertling & A. Zimmer, "Oligonucleotide and plasmid DNA packaging into polyoma VP1 virus-like particles expressed in *Escherichia coli", Biotechnol. Appl. Biochem.* 29, S. 31-43, 1999); auch hier ist die Effizienz und Spezifität des Einbaus aufgrund der ungericheten, statistischen Natur des Prozesses in Lösung nur gering.

Aufgabe der vorliegenden Erfindung ist es daher, die genannten Nachteile des Stands der Technik zu beseitigen.
Dies wird erfindungsgemäß durch ein Verfahren gemäß Anspruch 1 zum Einschluß von molekularen Substanzen in Proteinhüllen mit folgenden Schritten gelöst:
- Binden eines Proteinhüllenfragments über einen ersten Bereich an eine Matrix;
- Inkontaktbringen des an die Matrix gebundenen Proteinhüllenfragments mit der molekularen Substanz, um die molekulare Substanz an einen zweiten Bereich des Proteinhüllenfragments zu binden;
- Abtrennen des Proteinhüllenfragments mit der daran gebundenen molekularen Substanz, oder eines Teils des Proteinhüllenfragments, der die gebundene molekulare Substanz enthält, von der Matrix; und
- Assemblieren des abgetrennten Proteinhüllenfragments oder eines Teils hiervon, der die gebundene molekulare Substanz enthält, mit anderen Proteinhüllenfragmenten zu einer Proteinhülle.
wobei das Abtrennen und das Assemblieren in beliebiger Abfolge durchgeführt werden können.

Die vorliegende Erfindung betrifft auch eine Matrix mit einem daran gebundenen, assemblierbaren Proteinhüllenfragment gemäß Anspruch 20.

Vorteilhafte Ausgestaltungen des Verfahrens ergeben sich aus den Unteransprüchen und aus der Beschreibung.

### Beschreibung

Die vorliegende Erfindung betrifft die Herstellung von Transportsystemen, die unter anderem zum Gentransfer, Wirkstofftransfer und für die Gentherapie geeignet sind, die aus *in vitro* hergestellten Proteinhüllen abgeleitet sind. Bei Verfahren nach dem Stand der Technik wird ein Hüllproteintyp oder mehrere Hüllproteinspezies von - zumeist ikosaedrischen - Viren oder Phagen durch geeignete Wahl der Lösungsmittelbedingungen zur Assemblierung zu regelmä-ßigen Strukturen veranlaßt, die in ihrem Inneren einen geschützten Hohlraum umfassen. Der durch die Proteinhülle ausgebildete Hohlraum ist für den Transport molekularer Substanzen nutzbar. Im Falle klassischer Gentransfer-Anwendungen wird dazu ein geeignetes DNA-Plasmid, das für ein oder mehrere Gene kodiert, als die in Zellen zu transportierende Substanz verwendet. Die *in vitro*-Verpackung von Nukleinsäuren in virusanaloge Partikel wie z. B. in Polyomavirus-Panikel in Lösung ist bereits seit langem untersucht (vgl. S.N. Slilaty, K.I. Bems. & H.V. Aposhian, ..Polyoma-like particle: characterization of the DNA encapsidated *in vitro* by polyoma empty capsids", *J. Biol. Chem.* 257, S. 6571-6575, 1982). Neben Viren können grundsätzlich auch Phagen für diese artifizielle Verpackung herangezogen werden.

Das gezielte Einbringen von Wirkstoffmolekülen, insbesondere von DNA-basierten Expressionsplasmiden, in virusanaloge Partikel ist derzeit nach dem Stand der Technik ineffektiv.

Im Falle des Polyomavirus-Hüllproteins VP1 beispielsweise ist das gängige Verfahren zum Beladen der virusanalogen Partikel mit DNA die Verwendung einer osmotischen Schock-Prozedur, die jedoch ineffizient ist und bei der möglicherweise nur eine äußerliche Beladung der artifiziellen, virusähnlichen Kapside mit der DNA erfolgt. Die vorliegende Erfindung betrifft ein neuartiges Verfahren zur gerichteten Verpackung von molekularen Substanzen, beispielsweise von Nukleinsäuren, Peptiden, Proteinen oder anderen Wirkstoffen in das geschützte Innere von Proteinhüllen.

Das Verfahren, das in der vorliegenden Erfindung beschrieben wird, kann grundsätzlich zur gerichteten und hocheffizienten Verpackung von molekularen Substanzen verschiedenster Art in Hüllen, beispielsweise abgeleitet von Viren oder Phagen, verwendet werden. Der Einschluß solcher molekularen Substanzen kann für viele technische oder medizinischtherapeutische Prozesse genutzt werden.

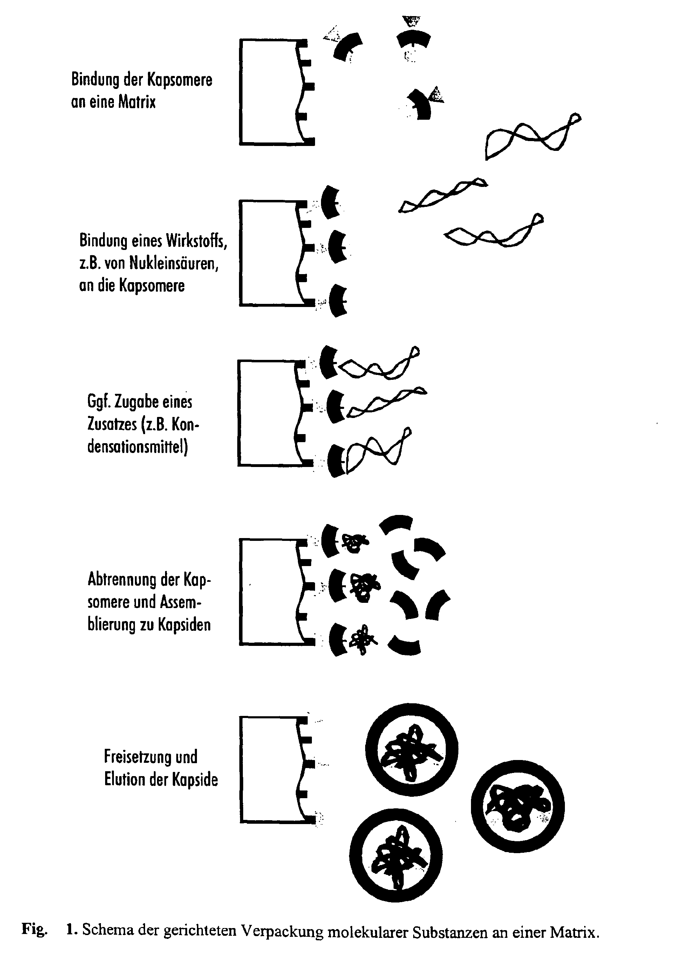

Bei dem erfindungsgemäßen Verfahren wird ein Bestandteil der Proteinhülle (in dieser Erfindung als Proteinhüllenfragment bezeichnet) mit einem ersten Bereich (Segment) an eine Matrix gebunden bzw. immobilisiert. Ein zweiter Bereich des Proteinhüllenfragments, an den die molekulare Substanz binden kann, ist dabei unfixiert und für die zu verpackende molekulare Substanz frei zugänglich. Das an die Matrix gebundene bzw. immobilisierte Proteinhüllenfragment wird nun mit der zu verpackenden molekularen Substanz in Kontakt gebracht. Anschließend wird die Abtrennung des beladenen Proteinhüllenfragments von der Matrix bewirkt. Zuvor, gleichzeitig, oder nachfolgend erfolgt eine Assemblierung oder Zusammenlagerung des beladenen Proteinhüllenfragments mit anderen Proteinhüllenfragmenten. Die molekulare Substanz kann bei diesem Verfahren gerichtet in die Hülle verpackt werden. Bei dem erfindungsgemäßen Verfahren können auch molekulare Substanzen verpackt werden, die in Lösung zur Aggregation neigen oder andere ungünstige Eigenschaften aufweisen. In Fig. 1 ist schematisch und beispielhaft eine mögliche Ausführungsform der Erfindung gezeigt, bei der als Proteinhüllenfragmente Virus-Kapsomere eingesetzt werden.
Als zu verpackende molekulare Substanzen können erfindungsgemäß einzelsträngige oder doppelsträngige DNA, einzelsträngige oder doppelsträngige RNA, Peptide, Peptidhormone, Proteine, Proteindomänen. Glykoproteine, Ribozyme, PNA (Peptide Nucleic Acid), pharmazeutische Wirkstoffe, z. B. mit hydrophilem oder hydrophobem oder amphiphilem Charakter, Nukleotide, Hormone, Lipide, oder Kohlenhydrate eingesetzt werden. Vorteilhaft sind DNA in Form von linearen oder zirkulären Plasmide, einzelsträngigen oder doppelsträngigen Oligonukleotiden, Chromosomen oder Chromosomenfragmenten, oder Proteine in Form von Antikörpern, single-chain-Antikörpern, Enzymen oder Markerproteinen, oder RNA in Form von antisense-RNA. Ribozymen, katalytischer RNA. oder codierender mRNA.

Bei Bedarf können weitere Zusätze, wie beispielsweise freie Untereinheiten des Hüllproteins, Varianten hiervon, oder Nukleinsäure-Kondensationsagentien, im Verlauf des Verfahrens verwendet werden.

Im Fall der Verpackung von DNA als molekulare Substanz, die in unkondensierter Form zu groß für eine Verpackung in Proteinhüllen ist, können erfindungsgemäß nach dem Inkontaktbringen des an die Matrix gebundenen Proteinhüllenfragments mit der molekularen Substanz ein oder mehrere Kondensationssubstanzen zugegeben werden, um eine kompaktere Struktur der molekularen Substanzen zu erreichen.

Das in der vorliegenden Erfindung beschriebene Verfahren zur Verpackung molekularer Substanzen ist nicht auf ikosaedrische Viren und Phagen als Proteinhüllen beschränkt, sondern kann auch auf morphologisch andersartig gestaltete Viren und Phagen sowie auf makromolekulare Assoziate mit innerem Hohlraum wie Proteasomen oder Chaperone angewandt werden. Die nachstehende Tabelle 1 zeigt die bezüglich ihrer Morphologie zusammengefaßten Viren und Phagen auf, die erfindungsgemäß eingesetzt werden können. Besonders gut untersucht ist hierbei die Gruppe der ikosaedrischen Viren.

**Tab. 1: Erfindungsgemäß als Proteinhüllen verwendbare Viren und Phagen**

| Morphologie | Virus bzw. Phage |
|---|---|
| Amorph bzw. | Umbravirus. Tenuivirus |
| Struktur unbekannt | |
| bacilliform | Baculoviridae; Badnavirus; Bamaviridae; Filoviridae; Rhabdoviridae |
| filamentös | Capillovirus; Carlavirus; Closterovirus; Furovirus; Inoviridae; Lipothrixviridae; Potexvirus; Potyviridae; Tobamovirus; Tobravirus; Polydnaviridae |
| helikal | Hordeivirus; Paramyxoviridae; Trichovirus |
| ikosaedrisch | Adenoviridae; Astroviridae; Bimaviridae; Bromoviridae; Caliciviridae; Caulimovirus; Circoviridae; Comoviridae; Corticoviridae; Dianthovirus; Enamovirus; Hepadnaviridae; Herpesviridae; Idaeovirus; Iridoviridae; Lviviridae; Luteovirus; Machlomovirus; Marafivirus; Microviridae; Necrovirus; Nodaviridae; Papovaviridae; Partitiviridae; Parvoviridae; Phycodnaviridae; Picornaviridae; Reoviridae; Rhizidiovirus; Sequiviridae; Sobemovirus; Tectiviridae; Tetraviridae; Tombusviridae; Totiviridae; Tymovirus |
| isometrisch | Cystoviridae; Geminiviridae |
| oval | Poxviridae |
| pleomorph | Coronaviridae; Hypoviridae; Plasmaviridae |
| sphärisch | Arenaviridae; Arterivirus, Bunyaviridae; Flaviviridae; Orthomyxoviridae; Retroviridae; Togaviridae |
| zitronenförmig | Fuselloviridae |
| Phagen mit | Myoviridae; Podoviridae; Siphoviridae |
| Schwanzforsatz | |

Ein Beispiel für solche Proteinhüllen ist nachstehend anhand des Polyomavirus-Pseudokapsids (aus VP1-Untereinheiten des Polyomavirus bestehende Proteinhülle) gezeigt.

Bei den in vorstehender Tabelle 1 dokumentierten Beispielen für verwendbare Viren und Phagen ist das SSV1-Partikel (Fuseolloviridae) hervorzuheben, das das Archaebakterium *Sulfolobus shibatae* infiziert. Dieser Vertreter der Phagen ist aufgrund seiner Wirtsspezifität hyperthermophil, mithin auch bei hohen Temperaturen stabil und kann daher für eine Vielzahl von Anwendungen im Bereich der Biotechnologie und auch der Medizin vorteilhaft genutzt werden. Es ist in der Lage, eine sehr stabile Proteinhülle auszubilden, wobei die Bausteine einfach rekombinant herstellbar sind. Ähnliche Vertreter thermophiler bzw. hyperthermophiler Phagen finden sich beispielsweise auch bei den Lipothrixviridae. Noch nicht weiter klassifiziert sind die thermophilen und hyperthermophilen Vertreter der Bacilloviridae und der Guttaviridae, die ebenfalls in solchen Prozessen, wo die Stabilität einer Proteinhülle (gebildet aus den Phagenproteinen) relevant ist, eingesetzt werden können.

Als Proteinhüllenfragmente können erfindungsgemäß monomere Teileinheiten oder Dimere oder Oligomere von Teileinheiten der oben genannten Proteinhüllen verwendet werden.

Die Proteinhüllenfragmente können erfindungsgemäß auf verschiedene Weise modifiziert sein, solange sie noch mit anderen Proteinhüllenfragmenten zu einer Proteinhülle assemblierbar sind.

Der erste Bereich mit dem die Proteinhüllenfragmente an die Matrix binden, kann beispielsweise ein C-teiminaler, ein N-teiminaler oder ein anderer Bereich des Proteinhüllenfragments, beispielsweise eine Loop-Region, sein. Vorteilhafterweise ist der erste Bereich so gelegen, daß er sich nach der Assemblierung auf der Außenseite der Proteinhülle befindet.

Der erste Bereich, mit dem die Proteinhüllenfragmente an die Matrix binden, kann beispielsweise ein C-terminaler, ein N-terminaler oder ein anderer Bereich des Proteinhüllenfragments, beispielsweise eine Loop-Region, sein. Vorteilhafterweise ist der erste Bereich so gelegen, daß er sich nach der Assemblierung auf der Außenseite der Proteinhülle befindet.

Der erste Bereich kann erfindungsgemäß so modifiziert sein, daß dieser erste Bereich eine verbesserte Bindungsaffinität zu der Matrix aufweist. Bei diesen Modifikationen kann es sich beispielsweise um die Fusion eines Peptid oder einer Proteindomäne, das bzw. die bekannte Bindungseigenschaften besitzt, mittels gentechnischer Methoden an das Proteinhüllenfragment handeln. Eine solches bindungsvermittelndes Molekül kann aber auch auf chemischem Wege, beispielsweise durch spezifische Biotinylierung, nach der Herstellung des Proteinhüllenfragmentes, kovalent angebunden werden. Beispiele für solche Modifikationen im ersten Bereich sind die Bindung von GST (Glutathion-S-Transferase) an glutathionhaltige Matrices, die Verwendung eines His-Tags zur Kopplung an Nickel-Chelat-Matrices, Chitin-Bindungs-Domänen in Verbindung mit Chitin-Matrices, Cellulose-Bindungs-Domänen in Verbindung mit Cellulose-Matrices, polyionische Peptidsequenzen in Verbindung mit entgegengesetzt geladenen Matrixoberflächen (peptidbeladene Matrices oder typische Matrices aus dem Bereich der Ionenaustausch-Chromatographie), WW-Domänen bzw SH3-Domänen in Verbindung mit prolinhaltigen Matrices, Polypriolinpeptide in Verbindung mit an der Matrix immobilisierten WW-Domänen oder SH3-Domänen, Antigene in Verbindung mit antikörperbeladenen Affinitätsmatrices (beispielsweise aus der Immuno-Affinitäts-Chromatographie), Lectine in Verbindung mit kohlenhydratbeladenen Matrices, oder Protein A, Protein M, Protein G, oder Protein Z in Verbindung mit immobilisierten Antikörpern. Die bindungsvermittelnden Moleküle können auch über Linkersegmente mit den Proteinhüllenfragmenten verbunden sein.

Der zweite Bereich der Proteinhüllenfragmente, an den die molekularen Substanzen binden können kann beispielsweise ein C-terminaler, ein N-terminaler oder ein anderer, nicht terminal gelegener Bereich des Proteinhüllenfragments, bevorzugt eine Loop-Region, sein. Vorteilhafterweise ist der zweite Bereich so gelegen, daß er sich nach der Assemblierung auf der Innenseite der Proteinhülle befindet.

Der zweite Bereich kann erfindungsgemäß so modifiziert sein, daß dieser zweite Bereich eine verbesserte Bindungsaffinität zu der molekularen Substanz aufweist. Bei diesen Modifikationen kann es sich beispielsweise um ortsspezifische Mutagenese eines Abschnittes des Proteinhüllenfragmentes, eine Fusion mit einem Proteinfragment oder die Insertion eines Proteinfragmentes (Proteindomäne) oder eines Peptides handeln. In gleicher Weise kann auch eine Modifizierung des Proteinhüllfragmentes durch chemische Modifikation mit molekularen Substanzen erfolgen, die eine geeignete Bindungsaffinität wie gewünscht aufweisen. Beispiele für die Modifikation des zweiten Bereiches sind die dortige Anbindung oder Einbringung von Substratanaloga zur Bindung von Enzymen, die Verwendung von Lectinen oder lectinartigen Domänen zur Bindung von Kohlenhydraten, die Verwendung der bakteriellen Proteine A, Protein G, Protein M, oder Protein Z zur unspezifischen Bindung von Antikörpern, die Verwendung von C 1-Komplexen des Komplementsystems zur Bindung von Antikörpern bestimmter Klassen, die Anbindung von Antigenen zur spezifischen Bindung von Antikörpern, die Verwendung von prolinhaltigen Peptiden zur Anbindung von WW-Domänen oder SH3-Domänen, die Verwendung von SH3-Domänen oder WW-Domänen zur Anbindung von prolinreichen Peptiden, die Verwendung von polyionischen Peptiden zur Anbindung von Peptiden oder chemischen Verbindungen (Polymere) mit der jeweils entgegengesetzten elektrischen Ladung, die Verwendung von Oligonukleotiden zur Anbindung von komplementären Nukleotidsequenzen, wie beispielsweise die Verwendung von Poly-T-Tags zur Bindung des Poly-A-Tails von eukaryontischer mRNA.

Weitere funktionelle Modifikationen in weiteren Bereichen des Proteinhüllenfragments sind erfindungsgemäß ebenfalls möglich.

Das Binden des ersten Bereichs des Proteinhüllenfragments an die Matrix kann reversibel oder irreversibel erfolgen. Erfindungsgemäß sind unter Bindung in diesem Zusammenhang kovalente Bindungen, ionische Bindungen, van-der-Waals-Bindungen oder andere Wechselwirkungen zwischen Proteinhüllenfragment und molekularer Substanz zu verstehen. Im Fall einer irreversiblen Bindung des Proteinhüllenfragments mit dem ersten Bereich an die Matrix wird nach Binden der molekularen Substanz an den zweiten Bereich des Proteinhüllenfragments nur ein Teil des Proteinhüllenfragments von der Matrix abgetrennt, der den zweiten Bereich des Proteinhüllenfragments umfaßt und der mit anderen Proteinhüllenfragmenten zu einer Proteinhülle assemblierbar ist.

Als Matrix kann erfindungsgemäß jede Matrix verwendet werden, an die Proteinhüllenfragmente anbindbar sind, beispielsweise eine Chitin-Matrix, Sepharose-Matrix, Dextran-Matrix oder Diethylaminoethyl-Matrix verwendet werden. Die Matrix kann beispielsweise eine feste Matrix sein oder in Form eines Gels vorliegen.

Das Abtrennen der Proteinhüllenfragmente oder der assemblierten Proteinhüllen von der Matrix erfolgt nach an sich bekannten Verfahren. Beispielsweise kann ein Linkersegment durch eine spezifische, z. B. enzymatische Spaltung durch Proteinasen, oder durch die Aktivierung eines Inteins vom Proteinhüllenfragment abgetrennt werden. Als weitere Möglichkeiten stehen beispielsweise eine Änderung der Salzkonzentration (lonenstärke), des pH-Wertes, der Temperatur, oder die Zugabe von Lösungsmitteladditiven (beispielsweise chaotrope Substanzen) oder spezifisch wirkenden Liganden, zur Verfügung.
Gemäß einer Ausführungsform der Erfindung wird ein Proteinhüllenfragment, z. B. eine Kapsomer-Untereinheit des Polyomavirus VP1-Kapsids, reversibel über den (in diesem Falle C-terminalen) Teil des Proteinhüllenfragments an einer festen Matrix immobilisiert. Ein speziell beschaffenes - in einem späteren Ausführungsbeispiel z. B. nukleinsäurebindendes - Segment am Aminoterminus des Kapsomers ist hierbei nicht fixiert und für die zu verpackende molekulare Substanz frei zugänglich. Das so immobilisierte Kapsomer wird nun mit der zu verpackenden Nukleinsäure inkubiert und diese an das Kapsomer gebunden. Anschließend erfolgt die Abtrennung des nukleinsäure-beladenen Kapsomers von der Matrix. Gleichzeitig oder nachfolgend wird die Assemblierung des nukleinsäure-beladenen Proteinbestandteils mit anderen Kapsomeren zu einer virusähnlichen Proteinhülle (Kapsid) induziert. Dabei wird unter geeigneten, definierten molaren Verhältnissen von beladenen und unbeladenen Kapsomeren eine maximale Ausbeute an Nukleinsäure-enthaltenden Kapsiden erreicht. Diese Verhältnisse können vom Fachmann beispielsweise wie folgt bestimmt werden: Es wird jeweils ein Experiment durchgeführt, bei dem die Konzentration der beladenen Kapsomere konstant bleibt, während die Konzentration der unbeladenen Kapsomere jeweils variiert wird. Als Maßgabe für den Bereich der Variation kann die Stöchiometrie des Kapsids herangezogen werden; im Falle der Polyomavirus-Hülle, die aus 72 Kapsomeren besteht, kann die Variation der Konzentrationsverhältnisse beispielsweise im Bereich von 1:71 bis 71:1 erfolgen. Die Auswertung dieser Experimente sollte eine Optimumskurve für die relativen Mengen von beladenen und unbeladenen Kapsomeren aufzeigen. Bei Bedarf können bei der Assemblierung beispielsweise freie Untereinheiten des Proteins oder funktionelle Varianten hiervon eingesetzt werden. Im Falle der DNA-Verpackung können zusätzlich Nukleinsäure-Kondensationsagentien eingesetzt werden.

Bei einer Ausführungsform des erfindungsgemäßen Verfahrens wird vor der Verpackung der entsprechenden molekularen Substanz, beispielsweise Nukleinsäuren, in die Proteinhülle des virusanalogen Kapsides ein Teil der noch nicht assemblierten Hülle (ein Proteinhüllenfragment) reversibel an eine Matrix immobilisiert. Ein zweiter Bereich des Proteinhüllenfragments, der eine ausreichend hohe Nukleinsäurebindungsfähigkeit (bzw. entsprechende Affinität zur jeweils gewählten molekularen Substanz) aufweist, ist dabei unfixiert und der gelösten Substanz frei zugänglich sein. Zu dem dergestalt immobilisierten Proteinhüllenfragment (Kapsomer) kann anschließend die molekulare Substanz (beispielsweise eine Nukleinsäure) gegeben werden, beispielsweise in Form eines üblichen ringförmigen Plasmids, das für ein Gen kodiert Diese Nukleinsäure bindet über das unfixierte Nukleinsäurebindungssegment an das immobilisierte Kapsomer, beispielsweise in der Stöchiometrie 1:1 (ein Nukleinsäuremolekül pro immobilisiertem Kapsomer). Erfindungsgemäß sind unter Bindung in diesem Zusammenhang kovalente Bindungen, ionische Bindungen, van-der-Waals-Bindungen oder andere Wechselwirkungen zwischen Proteinhüllenfragment und molekularer Substanz zu verstehen. Die so gebundenen Nukleinsäuren bzw. Wirkstoffe sind nun räumlich voneinander getrennt. Als besonderer Vorteil der Erfindung können die molekularen Substanzen aufgrund dieser räumlichen Separation nicht mehr miteinander wechselwirken bzw. aggregieren.

Im Falle der Verwendung von üblichen Wirkstoffen wie Proteinen, Peptiden, einzelsträngigen und ausreichend kurzen Oligonukleotiden, etc. kann danach unmittelbar der Elutionsschritt angeschlossen werden. Im Falle der Verwendung von komplexen und großen Wirkstoffen wie beispielsweise großen DNA-Plasmiden kann ein Zwischenschritt notwendig sein. Im Falle von DNA ist dies in der Regel die Verwendung eines Kondensationsagens. Beispielsweise wird durch die Verwendung von Histonen, histonähnlichen Proteinen, oder entsprechenden polykationischen Molekülen die Kompaktierung der fixierten DNA induziert. Die störende Bildung von Toroiden oder Aggregaten wird in diesem Fall durch die räumliche Separation und die indirekte Fixierung der Nukleinsäuremoleküle an der Matrix vermieden.

Zur Kondensation von DNA bei Verwendung derselben als zu verpackende molekulare Substanz oder Wirkstoff können im erfindungsgemäßen Verfahren grundsätzlich beliebige Kondensationsagentien verwendet werden, also beispielsweise Histone, histonähnliche Proteine, Polykationen, Polyarginin, Polylysin, Spermidin, methyliertes Spermidin, CTAB (Cetyltrimethylammoniumbromid) kationische Lipide, Lipospermin, Polyethylenglykol, Polyethylenimin, Kobalt-Amin-Verbindungen oder Manganverbindungen.

Durch einfache Versuche kann der Fachmann feststellen, ob das Kondensationsmittel mit der Bindung der Nukleinsäure an das Kapsomer konkurriert und dadurch die Nukleinsäure vorzeitig vom Kapsomer wieder abgetrennt wird. Analog gilt dies für alle verwendbaren Wirkstoffe.

Die komplette Proteinhülle kann in der Folge vollständig um den Wirkstoff bzw. die kondensierte Nukleinsäure herum assembliert werden, wobei das gebundene Kapsomer den Initiationsbaustein bildet und dazu definierte Mengen von freien Kapsomeren (oder Varianten hiervon) gegeben werden. Die Assemblierung kann wahlweise bereits während der Elution, also noch in der unmittelbaren räumlichen Umgebung der Matrix, oder nach der Freisetzung des Komplexes aus Kapsomer und Wirkstoff und Abtrennung von der Matrix durchgeführt werden. Für bestimmte Prozesse kann auch eine Assemblierung an der Matrix, noch vor der Freisetzung der Kapsomere, vorteilhaft sein; auch dies ist bei erfindungsgemäßer Anwendung möglich.

Das Kapsomer kann so ausgestaltet sein, daß sich an einem im Inneren des Kapsids liegenden Bereich (beispielsweise bei Verwendung des Protein PyVP1 als Kapsomer der N-terminale Teil) ein hochaffin nukleinsäurebindendes Segment befindet. Dies kann im Fall des PyVP1 der natürliche N-Terminus des Wildtypproteins sein, der bekanntermaßen nukleinsäurebindende Eigenschaften aufweist. In anderen Virus- oder Phagenhüllen kann dies auch der C-Terminus sein oder eine Loopstruktur im Kapsidinneren, die sich anhand der räumlichen Struktur des jeweiligen Hüllproteins erkennen läßt. Vorteilhaft ist die Verwendung von modifizierten Segmenten, die außergewöhnlich hochaffin DNA oder spezifisch andere Nukleinsäuretypen binden. Ein Beispiel dafür sind die gut untersuchten protamin-verwandte Sequenzen aus dem Kapsidprotein des Hepatitis B-Virus p21.5, die DNA- und RNA-Bindungseigenschaften aufweisen (vgl. T. Hatton, S. Zhou & D.N. Standring, "RNA- and DNA-binding activities in Hepatitis virus capsid protein: a model for their roles in viral replication", *J*. *Virology* 66, S. 5232-5241, 1992). In den nachfolgenden Beispielen sind demzufolge eine Reihe von N-terminal modifizierten Kapsomeren beschrieben, die solche hochaffinen Bindungssegmente aufweisen. Die erfindungsgemäße Anwendung soll jedoch nicht auf diese exemplarischen Beispiele beschränkt sein. Vielmehr sind im Rahmen der erfindungsgemäßen Anwendung viele weitere hochaffine Bindungssegmente einsetzbar. Für die Bindung von anderen Wirkstoffen gilt Entsprechendes; Proteine und Peptide können durch die Verwendung von affinen Bindungspartnern an die Kapsomerstruktur gebunden werden, wie dies in den nachfolgenden Beispielen demonstriert ist. Beispiele hierfür sind die Avidin-Biotin-Wechselwirkung, die Wechselwirkung von WW-Domänen bzw. von SH3-Domänen mit prolinreichen Peptidsequenzen, die Interaktion von entgegengesetzt geladenen polyionischen Sequenzen, etc.

Als besonders vorteilhaft für die Erfindung kann die Verwendung eines intein-basierten Fusionskonstruktes im beschriebenen Verfahren angesehen werden. Hier erfolgt die Abtrennung der immobilisierten Komplexe von der Matrix durch die Änderung der Redoxbedingungen in der Lösung, beispielsweise durch einfache Zugabe von Dithiothreitol (DTT) oder Hydroxylamin, mithin also durch Spaltung einer kovalenten Bindung. Diese Redoxbedingungen können nämlich einfach verändert werden, ohne daß die Anwesenheit von geladenen Kapsomeren oder Wirkstoffmolekülen wie z. B. DNA oder andere Nukleinsäuren dabei störend wirkt.

Erfindungsgemäß besonders vorteilhaft ist die Verwendung eines oligomeren Kapsomers, da die Matrixfixierung eines Oligomers (durch kooperative Effekte) erheblich stärker ist als die von einzelnen Monomeren.

Das Verfahren bietet weiterhin den Vorteil, daß an dem immobilisierten Bestandteil (vorzugsweise einem Kapsomer aus einer Proteinhülle) auch ein Screening zur Optimierung der Verpackungseigenschaften des Systems durchführbar ist. Ein solches Screening kann zur Verbesserung der physikochemischen oder Lösungsmittelbedingungen, der DNA-Sequenz, des molekularen Bindungsmoduls, u.a. führen, die beim Verpackungsprozeß genutzt werden. Weiterhin sind Eigenschaften der molekularen Substanz wie beispielsweise deren maximal erlaubte Größe für die Verpackung analysierbar. Ein solches Größenlimit ist gerade beim Einschluß von DNA in virusanaloge Vektorsysteme von großer Bedeutung. Die Bestimmung des Limits kann auf einfache Weise dadurch geschehen, daß dem immobilisierten Kapsomer eine bezüglich der Fragmentgröße heterogene DNA-Population zugesetzt wird. Diese heterogenen Fragmente können beispielsweise enzymatisch, durch Anwendung von Restriktionsenzymen oder durch Scheren von großen DNA-Molekülen hergestellt werden. Nach erfindungsgemäßer Anwendung des beschriebenen Verfahrens und nach Benzonase-Verdau der nichtverpackten oder peripher aufgelagerten DNA wird die in die virusanalogen Partikel geschützt verpackte DNA auf einem Agarosegel analysiert. Die größte, noch in Partikel verpackte DNA markiert das Größenlimit, das mit dem Verfahren und unter Verwendung der entsprechenden Proteinhülle verpackt werden kann.

Eine wesentlicher Vorteil des Verfahrens ist, daß das an die Matrix gebundene Proteinhüllenfragment von anderer chemischer oder physikalischer Natur sein kann als die übrigen, zur Proteinhüllbildung verwendeten Bausteine. Dies hat den besonderen Vorteil, daß eine gemischte Assemblierung der Proteinhüllen ermöglicht wird. Der immobilisierte Bestandteil hat die spezifische Funktion der Bindung und Internalisierung der zu verpackenden molekularen Substanz. Weitere Funktionen für die Herstellung eines Vektorsystems mit nahezu beliebigen Eigenschaften können in den übrigen Bestandteilen der Proteinhülle verankert sein. Die gemischte, mosaikartige Assemblierung erlaubt somit die Generierung von heterogenen Partikeln, wobei durch den immobilisierten Bestandteil die gerichtete Einbringung einer Substanz in den inneren Hohlraum gewährleistet werden kann.

Schließlich kann der immobilisierte Proteinbestandteil oder die zu verpackende molekulare Substanz durch eine Markierung wie z. B. ein fluoreszierendes oder radioaktives Molekül, ein spezifisch bindendes Peptidsegment (tagging), eine Biotinylierung oder andere chemische Markierung, oder auch auf eine andere Art markierbar und damit nachträglich - nach der Assemblierung zu virusähnlichen Proteinhüllen - identifizierbar sein. Dies hat den besonderen Vorteil, daß dadurch eine Bestimmung der spezifischen Veipackungseffizienz durch Analyse der zuletzt assemblierten Proteinhüllen durchführbar ist; die hergestellten Proteinhüllen, die die Markierung aufweisen, besitzen als Teil der Hülle das Kapsomer, das für die Verpackung der molekularen Substanz zuständig ist und damit bei Wahl geeigneter Bedingungen mit hoher Wahrscheinlichkeit auch die molekulare Substanz selbst. Demgegenüber können leere Proteinhüllen, also solche, die sich in der Lösung aus den zugesetzten übrigen Bestandteilen der Proteinhülle spontan und unter Ausschluß des mit der zu verpackenden molekularen Substanz beladenen Hüllbestandteils gebildet haben, unterschieden werden. Durch diese besondere Markierung (gegebenenfalls auch in Verbindung mit einem spezifischen Bindungssegment oder einer Biotinylierung) ist auch eine Selektierung der mit der molekularen Substanz beladenen Proteinhüllen von nichtbeladenen Proteinhüllen möglich, und damit die Herstellung homogener Populationen von virusanalogen Proteinhüllen mit jeweils verpackter molekularer Substanz möglich.

Die nachfolgend beschriebenen Beispiele zeigen Ausführungsformen der vorliegenden Erfindung, ohne daß hierduch der Schutzumfang der Erfindung eingschränkt werden soll.

### In den Beispielen und in der Beschreibung wird auf die folgenden Figuren Bezug genommen:

**Figur 1** zeigt schematisch und beispielhaft eine Ausführungsform der Erfindung. Ein Kapsomer aus einer Virushülle (Proteinhüllenfragment) wird reversibel mit einem ersten Bereich an einer Matrix immobilisiert und mit der zu verpackenden molekularen Substanz in Kontakt gebracht, die an einen zweiten Bereich des Kapsomers ("innenliegende", affine Strukturen) bindet. Das beladene Proteinhüllenfragment wird von der Matrix abgetrennt und die Assemblierung/Zusammenlagerung mit freien Kapsomeren zum virusanalogen Partikel induziert. Dadurch kann eine gerichtete Verpackung der molekularen Substanz ins Innere des Partikels erreicht werden.

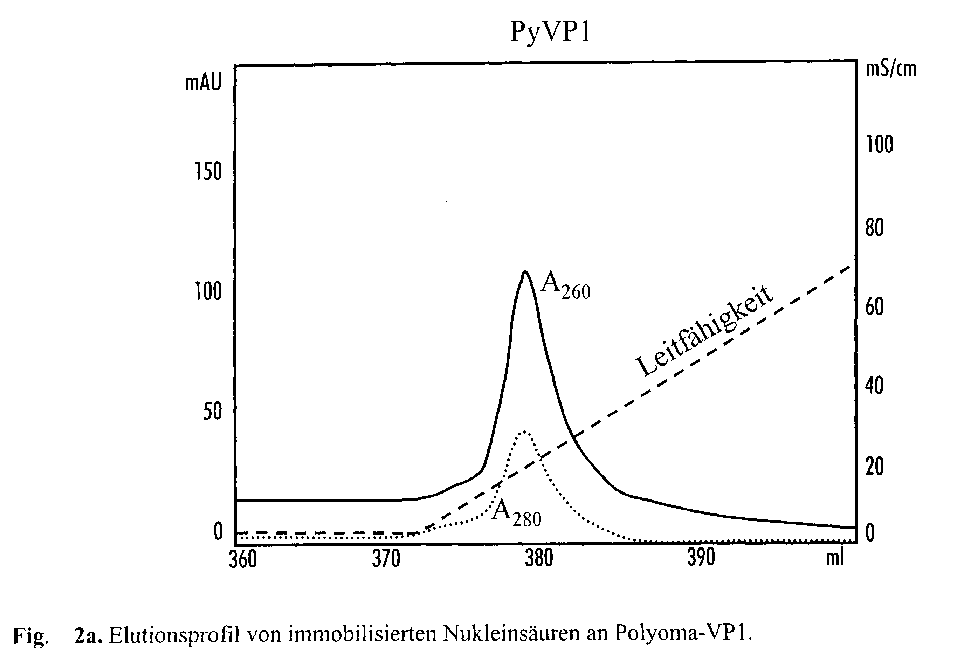

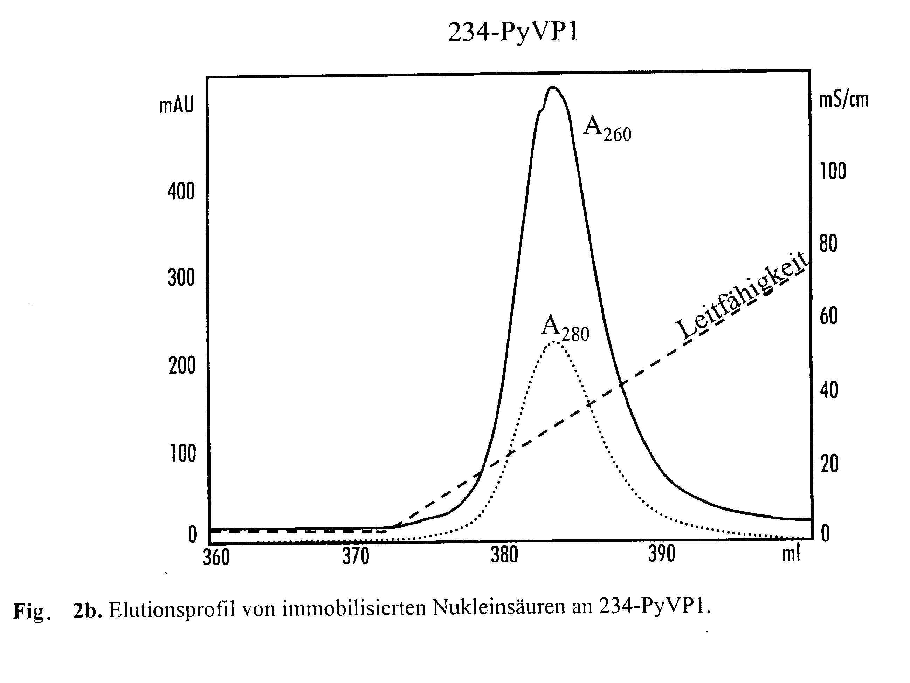

**Figur 2** zeigt Elutionsprofile von Nukleinsäuren (Plasmid pEGFP-N1), die zuvor an immobilisierte Kapsomere von VP1 (Fig. 2 a) bzw. 234-VP1 (Fig. 2 b) gebunden waren und durch einen linearen Salzgradienten zwischen 100 und 2000 mM NaCl von den Kapsomeren abgetrennt wurden. Die Elution der Nukleinsäuren von PyVP1-Wildtyp erfolgt bei 810 mM NaCl (Fig. 2a), die von 234-PyVP1 erfolgt bei 970 mM NaCl (Fig. 2b). 234-PyVP1 kann DNA somit noch stärker binden als Wildtyp-PyVP1.

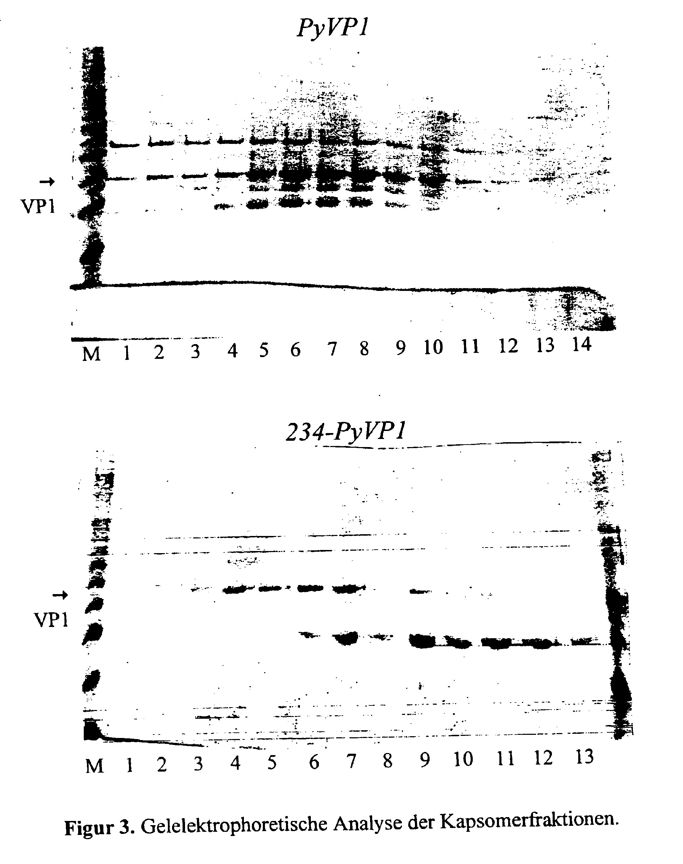

**Figur 3** stellt eine SDS-gelelektrophoretische Analyse (12 %iges Gel) des Proteingehaltes verschiedener Fraktionen nach Abspaltung der beladenen Kapsomere, Assemblierung zu Kapsiden, und Elution der beladenen Kapside von der Chitin-Matrix dar. Die Bahnen M bezeichnen jeweils den Molekularmassenstandard: die Laufhöhe des PyVP1-Proteins ist jeweils angezeichnet. Die Bahnen 1 bis 14 (bzw. 13) enthalten jeweils verschiedene Elutionsfraktionen des Versuchs.

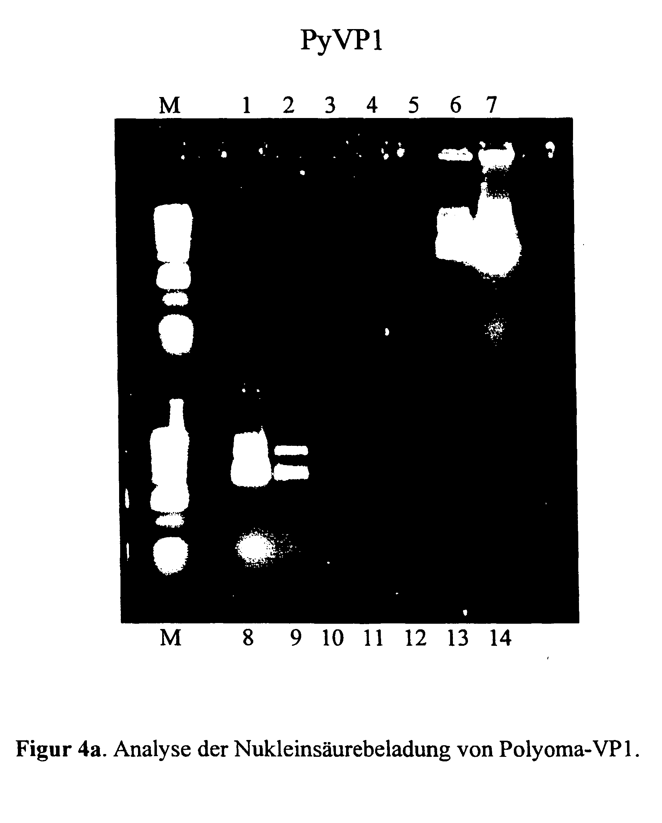

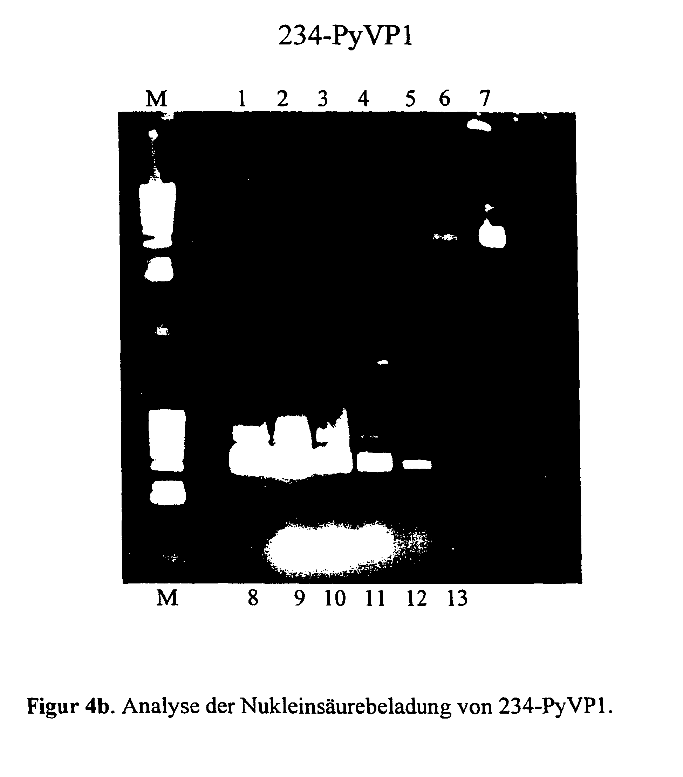

**Figur 4** zeigt die Analyse der Nukleinsäurebeladung der Kapsidfraktionen auf 1 %igen Agarosegelen, gefärbt mit Ethidiumbromid. Die Bahnen M bezeichnen den Größenstandard für Nukleinsäuren; Bahnen 1 bis 14 bei PyVP1 (Fig.4a) bzw. 1 bis 13 bei 234-PyVP1 (Fig. 4b) sind jeweils Elutionsfraktionen nach Extraktion der DNA mittels Standardprotokoll (Nummerierung der Bahnen ist identisch zur Figur 3). DNA befindet sich hauptsächlich in denselben Fraktionen, in denen nach Figur 3 auch Kapsidprotein vorhanden ist, das heißt, die DNA koeluiert zusammen mit dem Kapsid. Die 234-PyVP1-Variante (Fig. 4b) hat als besonderer Vorteil gegenüber dem PyVPI-Wildtypprotein (Fig. 4a) eine größere Menge an Nukleinsäuren verpackt, offenbar aufgrund der stärkeren DNA-Bindung des N-terminal angefügten protaminähnlichen Segmentes.

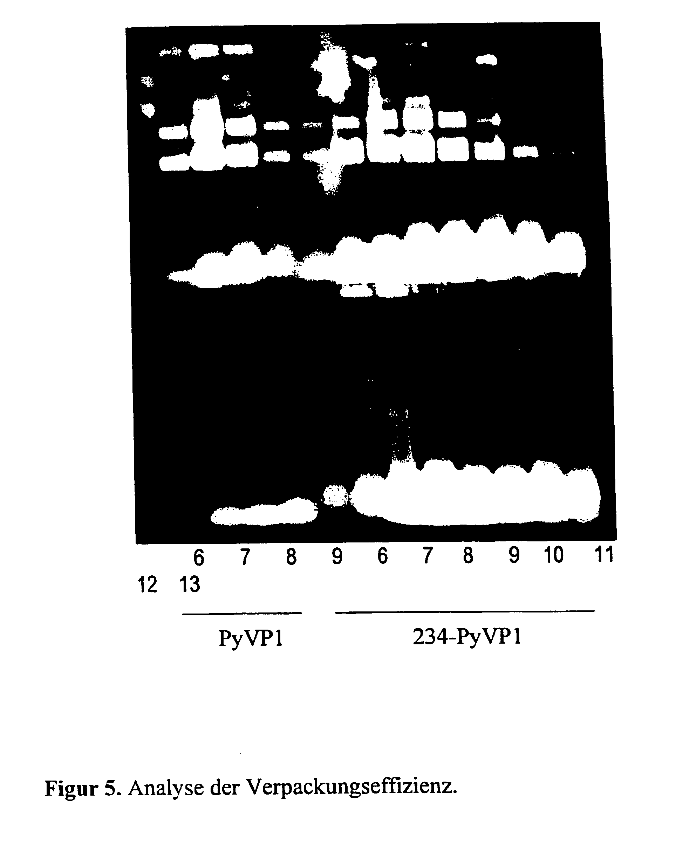

**Figur 5** zeigt die Analyse der Verpackungseffizienz der Kapside durch Verdau ungeschützter DNA mit Benzonase. Zu jeweils 400 µl der nach Matrixverpackung und Assemblierung über 2 Tage entstehenden Kapside von PyVP1 und 234-PyVP1 werden 2 µl Benzonase und 4 µl MgCl₂ (1 M) gegeben und der Ansatz für 1 h bei 37 °C inkubiert. Anschließend wird die extrahierte Nukleinsäure auf ein Agarosegel (1 %) aufgetragen und mit Ethidiumbromid gefärbt. Die obere Reihe der Figur stellen unbehandelte Kontrollproben dar, die untere Reihe zeigt die mit Benzonase behandelten Proben. Aufgetragen sind die Fraktionen 6 bis 9 von PyVP1-Kapsiden sowie 6 bis 13 von 234-PyVP1 (vgl. Figur 3 und 4). Die PyVP1-Kapside zeigen einen geringen Schutz vor Verdau, die 234-PyVP1-Kapside zeigen auch unter den gewählten stringenten Bedingungen noch einen deutlichen Schutz vor Nukleaseverdau, insbesondere in den Fraktionen 6 bis 8.

### Beispiel 1

### Herstellung von PyVP1-Kapsomeren mit Intein/CBD-Fusionsanteil zur Immobilisierung an einer Chitin-Matrix

Die Expression und Reinigung von PyVP1 erfolgt als Fusionsprotein mit einer C-terminal fusionierten Inteindomäne und einer sich daran anschließenden Chitin-Bindungsdomäne (CBD). Dazu wird zunächst ein Plasmid hergestellt, der auf dem kommerziell erhältlichen Vektor pCYB2 des IMPACT-Systems (Fa. New England Biolabs) beruht. Über die multiple Klonierungsstelle des pCYB2 wird über die Restriktionsschnittstellen Ndel - Xmal ein über PCR-Amplifikation hergestelltes und entsprechend mit den gleichen Restriktionsenzymen verdautes DNA-Fragment nach Standardverfahren einkloniert, das für das VP1-Gen von Maus-Polyomavirus codiert. Als Basis hierfür wird eine Polyomavirus-Variante verwendet, die keinerlei Cysteine in der Sequenz aufweist, die sechs Cysteine des Wildtyp-Proteins wurden zuvor durch Serin ersetzt. Diese Variante von PyVP1 hat den Vorteil, daß die Redoxbedingungen der Lösung keinen Einfluß auf den Zustand des Proteins haben, und ist dadurch in vielen Anwendungen einfacher handhabbar. Zu Markierungszwecken wurde bei dem Protein an der Stelle 249 anstelle eines Threonins ein neues Cystein eingeführt, das beispielsweise eine spezifische Markierung mit Fluoreszenzfarbstoffen ermöglicht, die verwendete Variante wird demzufolge mit PyVP1-CallS-T249C oder im Folgenden kurz PyVP1 bezeichnet. Für die PCR werden die folgenden Oligonucleotide als Primer verwendet: 5'-TAT ACA TAT GGC CCC CAA AAG AAA AAG C-3', und 5'-ATA TCC CGG GAG GAA ATA CAG TCT TTG TTT TTC C-3'. Bei dieser PCR werden gleichzeitig die C-terminalen Aminosäuren des Wildtyp-VP1-Proteins von Gly383-Asn384 umgewandelt in Pro383-Gly384, da ein C-terminal lokalisiertes Asparagin für das lntein-Spaltsystem sehr ungünstig ist. Die genannten Punktmutationen beeinflussen im Weiteren nicht die wesentlichen Eigenschaften des PyVP1-Proteins. Der tac-Promotor des pCYB2-Vektors liefert nur geringe Expressionsmengen des Fusionsproteins, daher wird das Fusionskonstrukt PyVP1-Intein-CBD über eine weitere PCR aus dem pCYB2-Vektor isoliert und in einen hochexprimierenden pET-Vektor- mit T71ac-Promotor (pET21a, Fa. Novagen), über NdeI - EcoRI - Restliktionsschnittstellen, kloniert. Oligonucleotide: 5'-TAT ACA TAT GGC CCC CAA AAG AAA AAG C-3', und 5'-ATA TGA ATT CCA GTC ATT GAA GCT GCC ACA AGG-3'.

Der entstandene Vektor erlaubt die Expression des Fusionsproteins PyVP1-Intein-CBD mit Hilfe des hochexprimierenden T71ac-Promotors in *E. coli* BL21(DE3)-Zellen (Fa. Novagen). Dazu werden die entsprechend transformierten Zellen in 5 1 - Erlenmeyerkolben, die je 2 1 LB-Medium enthalten, bei 37 °C angezüchtet, bis die OD₆₀₀ der Kultur 2.0 bis 2.5 beträgt. Zur Induktion der Proteinexpression wird IPTG zugesetzt bis zu einer finalen Konzentration von 1 mM. Die Kulturen werden zur Proteinexpression für weitere 20 Stunden bei 15°C inkubiert; die niedrige Temperatur verhindert dabei die Abspaltung des Intein-Anteils im Fusionsprotein unter *in vivo*-Bedingungen. Die Zellen werden anschließend durch Zentrifugation geerntet, in 70 ml Resuspensionspuffer (20 mM HEPES, 1 mM EDTA, 100 mM NaCl, 5 % (w/v) Glycerol, pH 8.0) aufgelöst, und durch Hochdruck-Homogenisation aufgeschlossen. Nach Zentrifugation des Rohextraktes für 60 min bei 48 000 g wird ein klarer Zellextrakt erhalten. Dieser wird unverzüglich mit einer Flußrate von 0.5 ml/min bei einer Temperatur von 10 °C auf eine 10 ml Chitin-Affinitätsmatrix (New England Biolabs) aufgetragen. Die Säule wird anschließend mit 3 Säulenvolumina des Resuspensionspuffers, 15 Säulenvolumina eines Waschpuffers hoher Ionenstärke (20 mM HEPES, 1 mM EDTA, 2 M NaCl, 5 % (w/v) Glycerol, pH 8.0) und wiederum 3 Säulenvolumina des Resuspensionspuffers gewaschen; dadurch werden alle unerwünschten *E*. *coli*-Wirtsproteine von der Chitin-Matrix entfernt.

An dieser Stelle des Experiments ist das PyVP1-Protein über den lntein-Fusionsanteil und der Chitin-Bindungsdomäne (CBD) mit hoher Affinität an der Chitin-Matrix immobilisiert und kann auch durch Puffer hoher Ionenstärke, z . B. dem vorstehend erwähnten Waschpuffer mit 2 M NaCl. nicht mehr von der Matrix eluiert werden. Als günstig hat es sich hierbei erwiesen, daß das PyVP1-Protein in Lösung ein Pentamer ist, das demzufolge als Fusionsprotein PyVPl-Intein-CBD mit 5 Bindungsstellen hochaffin an der Chitinmatrix fixiert wird. Der Fusionsanteil stört dabei nicht die Pentamerisierung des Proteins, verhindert aber die (vorzeitige) Assemblierung der Pentamere zu Kapsiden, für die ein freier C-Terminus des Proteins essentiell ist. Im vorstehend beschriebenen fixierten Zustand kann die Beladung des PyVP1-Pentamers über die freien N-Termini erfolgen (vgl. nachstehende Beispiele 2 und 3).

Durch einen Puls (3 Säulenvolumina) mit jeweils 50 mM Dithiothreitol (DTT). 50 mM Hydroxylamin, oder 30 mM DTT zusammen mit 30 mM Hydroxylamin, im Resuspensionspuffer kann die Abspaltung des PyVP1-Kapsomers aus dem Fusionsprotein vermittels der selbstspleißenden Inteinaktivität induziert werden. Dazu wird die beladene Chitinmatrix mit einer der angegebenen Lösungen für 14 Stunden bei 10°C inkubiert. Das PyVP 1 wird dabei vollständig freigesetzt und kann mittels säulenchromatographischer Verfahren von der Chitinmatrix und den an der Matrix anhaftenden übrigen Bestandteilen des Fusionsproteins eluiert und abgetrennt werden. Dazu wird geeigneterweise ein linearer Salzgradient mit einer Konzentration zwischen 0.1 und 2.0 M NaCl verwendet. Die Regeneration der Chitinmatrix erfolgt nach den Angaben des Herstellers durch Waschen des Chitin-Materials bei Raumtemperatur mit 3 Säulenvolumina eines SDS-haltigen Puffers (1 % SDS (w/v) in Resuspensionspuffer).

### Beispiel 2

### Bindung von Plasmid-DNA an matriximmobilisiertes PyVP1-Hüllprotein

Es ist bekannt, daß sich PyVP1 nach rekombinanter Expression, bei der es als pentameres Protein vorliegt, *in vitro* zu einer virusanalogen Proteinhülle assemblieren läßt. Auch die Assoziation von heterologer DNA an die Proteinhülle wurde bereits gezeigt, indem der Schutz der DNA vor Nuklease-Abbau gemessen wurde. Dies erfolgt allerdings mit nur geringer Ausbeute. Darüber hinaus wird ein Größenlimit von ca. 1 bis 2 kbp Länge für die geschützte DNA beobachtet.

Es wird vermutet, daß der N-Terminus des PyVP1-Proteins Nukleinsäurebindungseigenschaften aufweist. Diese natürlichen Bindungseigenschaften sind jedoch möglicherweise für einige Anwendungen nicht ausreichend. Daher wird alternativ zur Verwendung des Wildtyp-PyVP1 eine stark DNA-bindende Peptidsequenz, die abgeleitet ist aus der DNA-Bindungsregion des Kapsidproteins p21.5 des Hepatitis B-Virus (sogenannte Repeats II, III, und IV; nachfolgend als 234-Sequenz bezeichnet) an ein N-terminal um 18 Aminosäuren verkürztes PyVP 1 gentechnisch fusioniert. Dazu wird ein synthetisches doppelsträngiges 78-bp-Oligonukleotid der Sequenz 5'-ATG GCC AGC CCG CGT CGT CGT ACC CCA AGC CCA CGT CGT CGT CGT AGC CAG AGC CCG CGG TCG TCC GGT CGT AGC CAG-3' synthetisiert und über Standard-Klonierungsverfahren in den Expressionsvektor aus Beispiel 1 inseriert; dabei werden die ersten Aminosäuren des PyVP1-Proteins (Sequenz: MAPKRKSGVS KSETKSTK) durch die 234-Sequenz (codierende Sequenz des 78-bp-Oligonukleotides; Sequenz: MASPRRRTPS PRRRRSQSPR RRRSQ) ersetzt. Die Herstellung des 234-PyVP1-Proteins erfolgt analog den in Beispiel 1 angeführten Angaben. Überraschenderweise ist es gelungen, an auf diese Art immobilisiertes 234-PyVP1 eine Plasmid-DNA (pEGFP-N1, Fa. Clontech), stabil und definiert zu binden.

Im Standardpuffer mit niedriger Ionenstärke (20 mM HEPES, pH 8.0, 100 mM NaCl, 1 mM EDTA) bindet DNA nicht an die Chitinmatrix und erscheint nach Auftragen auf eine Chromatographiesäule mit Chitinmaterial im Durchlauf. Die Beladung der Chitinmatrix mit 234-PyVP1 wird gemäß den in Beispiel 1 beschriebenen Angaben durchgeführt. Nach dem Waschen der Säule mit einem Puffer hoher Ionenstärke (Standardpuffer wie oben, mit insgesamt 2.0 M NaCl) wird die Säule erneut mit Standardpuffer äquilibriert. Anschließend werden 30 µg des Plasmids injiziert. Dies entspricht 10 pmol des verwendeten Plasmids; bei einer Proteinmenge auf der Matrix von 2 - 4 mg (10-20 nmol 234-PyVP1-Pentamere) liegt das Plasmid etwa im tausendfachen molaren Unterschuß vor. Die DNA bindet unter diesen Bedingungen vollständig an die immobilisierten 234-PyVP1-Kapsomere. Ein identisches Experiment kann unter Verwendung des Wildtyp-PyVP1 durchgeführt werden und liefert zunächst analoge Ergebnisse.

Zur Analyse kann die DNA anschließend mit einem linearen NaCl-Gradienten zwischen 0.1 und 2.0 M NaCl wieder eluiert werden (vgl. Fig. 2). Das Elutionsmaximum der DNA liegt dabei im Falle des PyVP1 bei einer Salzkonzentration von 810 mM, bei Verwendung des modifizierten Proteins 234-PyVP1 dagegen bei einer Salzkonzentration von 970 mM NaCl, d.h. es liegt eine stärkere Bindung vor als beim Wildtyp-Protein. Dieses Experiment demonstriert, daß die DNA-bindende Eigenschaft von Wildtyp-PyVP1 und von 234-PyVP1 erhalten bleibt, wenn sie jeweils gemäß der vorstehenden Beschreibung als Fusionskonstrukte hergestellt werden und an eine feste Matrix immobilisiert werden. Gleichzeitig wird gezeigt, daß sich durch Wahl einer entsprechend affinen Bindungsstelle am N-Terminus von VP1 die Bindungseigenschaften für die zu verpackende molekulare Substanz modulieren lassen.

Analog zum beschriebenen Beispiel kann auch eine RNA-Bindungssequenz verwendet werden. Dazu wird anstelle der benannten 234-Sequenz der DNA-Bindungsregion des Kapsidproteins p21.5 des Hepatitis B-Virus das erste Repeat aus dieser Bindungsregion (Repeat I) verwendet, das repetitiv in drei Kopien hintereinander verwendet wird (111-Sequenz; Sequenz: MARRRDRGRS RRRDRGRSRR RDRGRS) und wiederum die ersten N-terminalen Aminosäuren des Wildtyp-PyVP1-Proteins ersetzen. Dazu wir das synthetische, doppelsträngige Oligonukleotid der Sequenz 5'-ATG GCG CGT CGT CGT GAT CGT GGC CGT AGC CGT CGT CGT GAT CGT GGT CGT AGC CGT CGT CGT GAT CGT GGT CGT AGC-3' über Standardverfahren in den entsprechenden Expressionsvektor kloniert und analog zum Beispiel 1 das resultierende 111-PyVP1-Protein hergestellt. Die repetitive Sequenz am N-Terminus bindet mit hoher Affinität RNA. Das auf gleiche Weise wie das 234-VP1 hergestellte 111-PyVP1-Protein weist damit analoge Eigenschaften auf wie das 234-PyVP1 mit der Ausnahme, daß spezifisch RNA anstelle von DNA als Nukleinsäure verpackt werden kann.

### Beispiel 3

### Koelution von DNA und VP1 nach Abspaltung vom Intein-Fusiosanteil

Der unter Beispiel 2 beschriebene Ansatz (unter Verwendung von 234-PyVP1) wird nach dem Auftrag der Nukleinsäure und der vollständigen Bindung derselben an die immobilisierten Kapsomere mit dem Spaltpuffer versetzt, der die Ablösung der Kapsomere vom Fusionsanteil induziert. Der Spaltpuffer enthält die Bestandteile des Standardpuffers, zusätzlich 50 mM DTT oder 50 mM Hydroxylamin (vgl. Beispiel 1) zum Spalten des Intein-Fusionskonstruktes nach Herstellerangaben (Fa. New England Biolabs), das Kondensationsmittel Cetyltrimethylammoniumbromid (CTAB) in einer Konzentration von 20 µM, und 0.5 mM CaCl₂ zur Induktion der Assemblierung der Proteinhülle des virusanalogen Kapsids aus den Kapsomeren. Nach Inkubation des Ansatzes auf der Säule bei 15°C über Nacht wird mit einem linearen NaCl-Gradienten eluiert und die separierten Fraktionen auf drei Arten analysiert.
- ***Protein**:* Ein mit Coomassie-Farbstoff gefärbtes SDS-Polyacrylamidgel wird zum Nachweis des eluierten Proteins. d.h. der freigesetzten Kapsomere, verwendet (vgl. Fig. 3).
- ***Nukleinsäure**:* Nach Phenol-Chloroform-Extraktion und Ethanolpräzipitation der DNA wird in einem mit Ethidiumbromid gefärbten Gel der DNA-Gehalt der Fraktionen bestimmt (vgl. Fig. 4).
- ***Geschützte (verpackte) Nukleinsäure**:* Nach Benzonaseverdau (Fa. Merck, 10 units in 400 µl), der durch Zugabe auf 0.5 % SDS und 20 mM EDTA und anschließender Phenol-Chloroform-Extraktion und Ethanolpräzipitation gestoppt wird, wird der Anteil an DNA, der vor Benzonaseaktivität geschützt ist (also ins geschützte Innere der assemblierten Proteinhülle aufgenommen wurde), bestimmt. Dies erfolgt durch Analyse auf einem mit Ethidiumbromid gefärbten Agarose-Gel (vgl. Fig. 5).

Die Auswertung des Versuches sowie Fig. 3 bis 5 zeigen, daß eine Koelution der DNA mit dem Protein erfolgt, d. h. die zugegebene DNA bindet stabil an die immobilisierten Kapsomere. Während der Spaltung und im Verlauf des Anlegens des linearen Salzgradienten zur Elution der Komplexe bleibt die Bindung zwischen den Kapsomeren und der DNA erhalten. Weiterhin wird ein erheblicher Schutz der DNA in einem Großteil der Fraktionen vor Abbau durch Nukleasen gezeigt. Dieser Schutz erstreckt sich auf die vollständige ursprünglichen Plasmidgröße, das heißt, das Plasmid wird jeweils komplett in die Kapside verpackt und dadurch vor dem Abbau durch Nuklease (Benzonase) geschützt. Im gezeigten Beispiel beträgt die Effizienz im Falle der 234-PyVP1-Kapside unter geeigneten Bedingungen etwa 20 % der eingesetzten DNA-Menge. In Verpackungsversuchen ohne Matriximmobilisierung nach dem Standardverfahren des osmotischen Schocks wurden dagegen maximal Effizienzen im Bereich von 2 - 4 % beobachtet. Das dargestellte Verfahren ist somit wesentlich dazu geeignet, eine gerichtete Verpackung von molekularen Substanzen wie z.B. Nukleinsäuren in das Innere einer Proteinhülle zu bewerkstelligen.

### Beispiel 4

### Transfektion von matrixverpackter DNA in eukaryontische Zellen

Das in Beispiel 3 beschriebene System wird zum Gentransfer des Plasmids pEGFP-N1 in NIH3T3-Zellen verwendet Die nach Elution von der Säule fraktionierten Kapside werden dazu in Gegenwart von Benzonase (5 units) zum Abbau noch vorhandener, freier Plasmid-DNA zu NIH3T3-Zellen gegeben, die am Vortag ausgesät werden (20 000 Zellen und 40 µg Komplex pro Ansatz). Nach 48 h Inkubation der Zellen bei 37 °C und 5 % CO₂ werden die Zellen dreimal mit PBS gewaschen, 5 min mit Trypsin/EDTA-Lösung inkubiert und anschließend vom Untergrund abgelöst. Bei der Expression des EGFP-Gens wird das grün fluoreszierende Protein GFP produziert, dessen Fluoreszenz im Fluoreszenz-aktivierten Zellsortierer (FACS) gemessen werden kann. Eine Analyse der transfizierten Zellen im FACS-Gerät zeigt eine GFP-Gesamtfluoreszenz die 170 % der Grundfluoreszenz von nicht transfizierten Zellen beträgt. Damit ist gezeigt, daß die an der Matrix nach den Beispielen 1 bis 3 hergestellten virusanalogen Kapside in der Lage sind, effizient molekulare Substanzen wie z. B. Nukleinsäuren in Zellen zu transportieren. Die verwendeten Komponenten sind dabei nicht toxisch; in den Zellen kann die Nukleinsäure zur Expression gebracht werden. Es findet durch das verwendete System also ein produktiver Gentransfer statt.

### Beispiel 5

### Verpackung von Peptiden, Proteinen, oder pharmazeutischen Wirkstoffen

Neben der in den Beispielen 2 bis 4 beschriebenen Verwendung von Nukleinsäuren kann auch eine gerichtete Verpackung von anderen Wirkstoffen, wie beispielsweise Proteinen oder Peptiden, erfolgen. Dazu wird die Bindungsstelle an der Innenseite des Kapsomers so modifiziert, daß eine Anlagerung des Protein- oder Peptidwirkstoffs über eine affine Bindung an diese modifizierte Bindungsstelle erfolgt. Diese Anbindung kann beispielsweise durch ionische Wechselwirkungen, das heißt, durch zwei entgegengesetzt geladene Peptidabschnitte, erfolgen, oder es kann eine Bindung über Wasserstoffbrücken zwischen dem Kapsomer auf seiner Innenseite und dem Peptid- oder Proteinwirkstoff erfolgen. Schließlich können auch natürliche, bekannte Wechselwirkungen wie die zwischen SH3-Domänen und prolinreichen Sequenzen, oder die zwischen dem Protein Avidin und der als Strep-Tag bekannten Peptidsequenz verwendet werden. Dabei wird einer der Bindungspartner bei der Herstellung mit einem entsprechenden Bindungssegment versehen, der zu verpackende Wirkstoff analog bei seiner Herstellung mit dem komplementären Bindungssegment. Die Anbindung der Bindungssegmente kann bei rekombinanter Herstellung der Kapsomere mit Hilfe von gentechnischen Standardverfahren besonders einfach erfolgen, wenn Peptide, Proteine, oder Proteindomänen als Bindungssegmente verwendet werden. Diese Bindungssegmente können nach Standardverfahren produziert werden. In gleicher Weise gilt dies für den zu verpackenden Wirkstoff mit dem daran befindlichen komplementären Bindungssegment. Nach der Immobilisierung des Kapsomers an der Matrix und der erfolgten Anbindung des Peptid- oder Proteinwirkstoffs unter Vermittlung der beiden komplementären Bindungssegmente erfolgen wiederum die Schritte der Abtrennung von der Matrix und die Assemblierung der Kapsomere zur Proteinhülle, wobei die Reihenfolge der beiden Schritte je nach Prozeß variieren kann. Auf diese Weise kann in Analogie zur vorstehend beschriebenen Nukleinsäureveipackung auch eine gerichtete Verpackung anderer Wirkstoffe, wie zum Beispiel Proteine oder Peptide, durchgeführt werden.

### Beispiel 6

### Verpackung von GFP (Green Fluorescent Protein)

In diesem Beispiel wird gezeigt, daß durch günstige Positionierung von Adaptersegmenten eine Dirigierung von molekularen Substanzen, in diesem Fall von Proteinen, in das Innere von Virusüllen oder von virusähnlichen Hüllen (Kapsiden) mit Hilfe des matrixgestützten Verfahrens erfolgen kann. Aufgrund der dreidimensionalen Struktur von Polyomavirus VP1 ist nach dem Stand der Technik bekannt, daß der N-Terminus des Proteins nach der Assemblierung zum Kapsid im Innenraum der Hülle lokalisiert ist. Es wird daher eine Variante des PyVP1-Proteins hergestellt, die eine sogenannte WW-Domäne am Aminoterminus des nativen Wildtyp-Proteins enthalten (Variante PyVP1-WW1). Basis für diese Variante ist wie in Beispiel 1 ein VP1-Protein von Polyomavirus, das alle natürlichen Cysteine durch Serine ersetzt hat und in das zusätzlich ein spezifisches neues Cystein eingeführt ist (PyVP1-CallS-T249C, abgekürzt PyVP1). WW-Domänen sind kleine Proteindomänen, die eine hohe Affinität zu prolinreichen Sequenzen besitzen: die WW-Domäne der Sequenz Gly-Ser-Gly-Trp-Thr-Glu-His-Lys-Ser-Pro-Asp-Gly-Arg-Thr-Tyr-Tyr-Tyr-Asn-Thr-Glu-Thr-Lys-Gln-Ser-Thr-Trp-Glu-Lys-Pro-Asp-Asp (aus dem FBP11-Genprodukt der Maus) zeigt dabei besonders hohe Affinität zur Peptidsequenz Pro-Pro-Leu-Pro.

Zunächst wird die Amplifikation einer WW-Domäne mittels PCR durchgeführt; als Templat dient dabei das FBP11-Gen der Maus. Als Oligonukleotide für die PCR werden dabei 5'-AAT ATA TCA TAT GTC CAT CAT CCG GCT TTT CCC AGG TAG ACT-3' (mit Ndel-Schnittstelle), und 5'-TAT TAA TCA TAT GAG CGG CTG GAC AGA ACA TAA ATC ACC TGA TGG-3' verwendet. Das somit erhaltene PCR-Produkt wird anschließend über die mittels der Oligonukleotide eingeführten Schnittstellen Nde I - Nde I in den Expressionsvektor pET21a aus Beispiel 1 einkloniert, der das Gen für ein Fusionsprotein PyVPI-Intein-CBD enthält, am 5'-Ende des Gens befindet sich dabei eine singuläre Nde I - Schnittstelle. Das exprimierte Genprodukt dieses Vektors ist das gewünschte Protein PyVP1-WW1. Expression und Reinigung des Proteins erfolgt in Übereinstimmung mit Beispiel 1. Dabei wird wie im Beispiel 1 das Protein an der Chitinmatrix immobilisiert und die nichtgebundenen Bestandteile durch die Waschlösung entfernt.

In analoger Weise wird die Herstellung und Reinigung einer GFP-Variante vorgenommen. GFP ist ein Protein, das im nativen Zustand eine grüne Fluoreszenz zeigt (Absorptionsmaximum bei 490 nm). Es eignet sich somit hervorragend zur Markierung von Komplexen und Assoziaten. Zur Herstellung einer GFP-Variante mit einer prolinreichen terminalen Sequenz erfolgt zunächst eine PCR-basierte Amplifikation des GFP-Gens, wobei als Templat das Plasmid pEGFP-N 1 (Fa. Clontech) verwendet wird. Gleichzeitig werden geeignete Restriktionsschnittstellen in das PCR-Produkt eingeführt. Die PCR erfolgt mittels der Oligonukleotide 5'-TTA TTT ACA TAT GGT GAG CAA GGG CGA GGA G-3' (mitNde I-Schnittstelle), und 5'-ATA TCT TAA GTA CAG CTC GTC CAT GCC G-3' (mit Afl II-Schnittstelle). Das so erhaltene PCR-Produkt wird über die Restriktionsschnittstellen in den Vektor pTIP kloniert und dort exprimiert; dieser Vektor pTIP ist ein Derivat des in Beispiel 1 dokumentierten Intein-Reinigungsvektors auf Basis von pET21a, mit zusätzlich eingeführten prolinreichen Sequenzen. Der Vektor ist dabei so konstruiert, daß wahlweise am 5'- oder 3'-Ende eines über eine multiple Klonierungsstelle eingefügten Gens eine prolinreiche Sequenz anfusioniert wird. Die prolinreiche Sequenz enthält dabei vorwiegend Pro-Pro-Pro-Pro-Pro-Pro-Pro-Pro-Leu-Pro, und damit die stark bindende prolinreiche Sequenz, die für die Anheftung an die WW-Domäne erforderlich ist. Die Herstellung und Reinigung des GFP-PPLP-Proteins erfolgt mittels Chitinaffinitätschromatographie, in inhaltlicher Übereinstimmung mit dem Beispiel 1 . Das GFP-PPLP-Protein, das am C-Terminus die prolinreiche Sequenz Pro-Pro-Pro-Pro-Pro-Pro-Pro-Pro-Leu-Pro trägt, kann löslich in großer Menge hergestellt werden. Die grün leuchtende Farbe der Proteinlösung zeigt gleichzeitig, daß das Protein zu seiner nativen Struktur falten kann.

Das PyVP1-WW1-Protein. das auf der Chitinsäule immobilisiert ist, wird zunächst mit dem GFP-PPLP (im molaren Verhältnis 1 : 6) auf der Matrix für 20 min inkubiert (Lösungsmittel: 10 mM HEPES, 1 mM EDTA. 150 mM NaCl, 5 % Glycerol, pH 7.2). Anschließend wird die Spaltung des PyV P1-WW 1-Fusionsproteins von der lnteindomäne durch Zugabe von Reduktionsmitteln gemäß Beispiel 1 induziert. Das PyVP1-WW I-Protein wird zusammen mit dem über die WW-Polyprolin-Affinität angebundenen GFP-Protein von der Säule eluiert und unmittelbar danach die Lösung mit unmodifizierten PyVP1-Proteinen im stöchiometrischen Verhältnis 1:1 bis 1: 10 versetzt. Die Kapsidbildung der PyVP1-Varianten wird durch Dialyse gegen einen Puffer induziert, der 0.5 mM CaCl₂ enthält. Anhand von Gelfiltrationsuntersuchungen (Säule Säule TSKGel G6000PWXL, Fa. TosoHaas) kann gezeigt werden, daß ein Teil des nativen GFP-PPLP-Proteins (identifizierbar anhand der spezifischen Absorption bei 490 nm) in der Kapsidfraktion (bei Elutionsvolumina zwischen 9 und 10 ml) enthalten ist. Das bedeutet, daß während der Inkubation des GFP-PPLP-Proteins mit der matrixassoziierten Variante PyVP1-WW1 auf der Säule eine Bindung der beiden Proteine aneinander erfolgte, wodurch das GFP bei der nachfolgenden Kapsidassemblierung in das Innere des virusähnlichen Partikels dirigiert wurde.

Zusammenfassend zeigt das Experiment in diesem Beispiel, daß Varianten von PyVPl mit N-terminal anfusionierter WW-Domäne dazu in der Lage sind, im matrixassoziierten Zustand prolinreiche Sequenzen zu binden und diese sowie die ggf. daran befindlichen molekularen Substanzen bei einer Assemblierung zu Kapsiden unter geeigneten Bedingungen in das Innere von virusähnlichen Hüllen zu dirigieren. Damit ist das beschriebene Verfahren dazu geeignet, eine gerichtete Verpackung von molekularen Substanzen in Viren bzw. in virusähnliche Kapside zu bewirken. Als wesentlicher Vorteil gegenüber der Verpackung in Lösung ist auch hier die Vermeidung von Aggregation der zu verpackenden Substanz zu nennen, die in ausreichend geringer Konzentration verwendet werden kann. Durch die Immobilisierung nach Bindung an das matrixfixierte PyVP1-WW1-Protein kann die Aggregation vollständig unterbunden werden, und die molekulare Substanz wird an der Säule aufkonzentriert, ohne daß eine Aggregation zum Tragen kommt.

### Beispiel 7

### Beladung über Biotin / Avidin (Streptavidin) - Wechselwirkung

Ein weiterer Anwendungsfall ist auch der Einsatz des verbreiteten Systems zur Herstellung von Wechselwirkungen auf der Basis von Avidin (oder Streptavidin bzw. anderer Derivate) mit Biotin. Diese Wechselwirkung ist außerordentlich stark; Moleküle, die miteinander über diese Verankerung wechselwirken, sind nahezu untrennbar miteinander verbunden. Diese Eigenschaft wird häufig zur Kopplung von Biomolekülen ausgenutzt. Ein Nachteil des Systems ist, daß Avidin insgesamt über vier Bindungsstellen für Biotin verfügt. Die einfache Inkubation von biotinylierten Substanzen mit Avidin führt daher oft zur unerwünschten Ausbildung von (Homo-)Tetrameren in Lösung, oder zur Bildung von Aggregaten. Im Rahmen der vorliegenden Erfindung kann dieser Nachteil nach dem Stand der Technik durch die Matriximmobilisierung aufgehoben werden. Dazu wird - wie in den vorstehenden Beispielen ausgeführt - beispielsweise ein modifiziertes virales Hüllprotein verwendet, das ein einziges singuläres Cystein an einer exponierten Stelle besitzt (im Beispiel 1 PyVP1-CallS-T249C, vgl. Sequenzprotokoll). Dieses Hüllprotein kann, wie in den vorstehenden Beispielen beschrieben, an einer Matrix immobilisiert werden. Anschließend wird ein Reagens zugegeben, das auf einer Seite eine reaktive Maleimidgruppe trägt, auf der anderen Seite eine funktionelle Gruppe wie beispielsweise Biotin; die beiden Gruppen werden dabei von einem angemessen langen Spacer voneinander separiert. Derartige Reagentien sind kommerziell erhältlich, beispielsweise bei Firma Pierce. Wird dieses Reagens nun mit dem immobilisierten PyVP1-CallS-T249C inkubiert, dann bildet die Maleimidgruppe eine stabile und spezifische kovalente Bindung mit dem singulären Cystein des PyVP1-CallS-T249C aus. Nachfolgend kann Avidin bzw. Streptavidin zugegeben werden, das mit hoher Affinität an die Biotin-Gruppe bindet. Anschließend kann das Kapsomer nach dem in Beispiel 1 beschriebenen Verfahren abgetrennt werden, und man erhält Kapsomere, die Avidin bzw. Streptavidin tragen. Diese können vor oder nach der Abtrennung von der Matrix noch mit einer weiteren biotinylierten Substanz inkubiert werden, wodurch man Kapsomere erhält, die stabil über die Biotin-Avidin-Biotin-Wechselwirkung eine beliebige molekulare Substanz gebunden hat, die beispielsweise im Inneren der Kapside verpackt werden kann. Durch das beschriebene matrixgestützte Verfahren wird vermieden, daß es zur Aggregatbildung oder zur frühzeitigen Bildung von Homotetrameren kommt, und es wird eine gleichförmige Beladung der Kapsomere bewirkt.

## Patentansprüche

1. Verfahren zum gerichteten Einschluß von molekularen Substanzen nämlich einzelsträngige oder doppelsträngige DNA, einzelsträngige oder doppelsträngige RNA, Peptide, Peptidhormone, Proteine, Proteindomänen, Glykoproteine, Ribozyme, PNA (Peptide Nucleic Acid), pharmazeutische Wirkstoffe, Nukleotide, Hormone, Lipide, oder Kohlenhydrate oder eine Kombination einer oder mehrerer dieser Substanzen in Proteinhüllen mit folgenden Schritten:
- Binden eines Proteinhüllenfragments mit einem ersten Bereich an eine Matrix, an die Proteinküllenfragmente anbindbar sind,
- Inkontaktbringen des an die Matrix gebundenen Proteinhüllenfragments mit der molekularen Substanz, um die molekulare Substanz an einen zweiten Bereich des Proteinhüllenfragments zu binden;
- Abtrennen des Proteinhüllenfragments mit der daran gebundenen molekularen Substanz, oder eines Teils des Proteinhüllenfragments, der die gebundene molekulare Substanz enthält, von der Matrix;
und
- Assemblieren des abgetrennten Proteinhüllenfragments oder eines Teils hiervon, der die gebundene molekulare Substanz enthält, mit anderen Proteinhüllenfragmenten zu einer Proteinhülle,
wobei das Abtrennen und das Assemblieren in beliebiger Abfolge durchgeführt werden können.

2. Verfahren nach Anspruch 1,
**dadurch gekennzeichnet,**
**daß** das Abtrennen und Assemblieren gleichzeitig erfolgen oder zunächst das Assemblieren und anschließend das Abtrennen erfolgt.

3. Verfahren nach Anspruch 1 oder 2,
**dadurch gekennzeichnet,**
**daß** nach dem Inkontaktbringen des an die Matrix gebundenen Proteinhüllenfragments mit der molekularen Substanz ein oder mehrere Kondensationssubstanzen zugegeben werden, um eine kompaktere Struktur der molekularen Substanzen zu erreichen.

4. Verfahren nach einem oder mehreren der vorhergehenden Ansprüche,
**dadurch gekennzeichnet,**
**daß** als Kondensationssubstanzen Histone, histonähnliche Proteine, Polykationen, Polyarginin, Polylysin, Spermidin, methyliertes Spermidin, CTAB (Cetyltrimethylammoniumbromid), kationische Lipide, Lipospermin, Polyethylenglykol, Polyethylenimin, Kobalt-Amin-Verbindungen oder eine Kombination einer oder mehrerer dieser Substanzen. verwendet werden.

5. Verfahren nach einem oder mehreren der vorhergehenden Ansprüche,
**dadurch gekennzeichnet,**
**daß** die molekulare Substanz DNA in Form von linearen oder zirkulären Plasmiden, einzelsträngigen oder doppelsträngigen Oligonukleotiden, Chromosomen oder Chromosomenfragmenten ist, oder
RNA in Form von antisense-RNA, Ribozymen, katalytischer RNA, oder codierender mRNA, oder
als molekulare Substanzen Proteine in Form von Antikörpern, single-chain-Antikörpern, Enzymen, Strukturproteinen, oder Markerproteinen eingesetzt werden.

6. Verfahren nach einem oder mehreren der vorhergehenden Ansprüche,
**dadurch gekennzeichnet,**
**daß** die Proteinhülle ein Viruskapsid, ein Phagenkapsid, oder ein makromolekulares Proteinassemblat mit innerem Hohlraum, bevorzugt ein Proteasom oder ein Chaperon-Komplex, ist.

7. Verfahren nach einem oder mehreren der vorhergehenden Ansprüche,
**dadurch gekennzeichnet,**
**daß** die Proteinhülle aus gleichen oder voneinander verschiedenen Proteinhüllenfragmenten besteht.

8. Verfahren nach einem oder mehreren der vorhergehenden Ansprüche,
**dadurch gekennzeichnet,**
**daß** das Proteinhüllenfragment ein Kapsomer von einem Virus oder einem Phagen der Adenoviridae, Arenaviridae, Arterivirus, Astroviridae, Bacilloviridae, Baculoviridae, Badnavirus, Barnaviridae, Bimaviridae, Bromoviridae, Bunyaviridae, Caliciviridae, Capillovirus, Carlavirus, Caulimovirus, Circoviridae, Closterovirus, Comoviridae, Coronaviridae, Corticoviridae, Cystoviridae, Dianthovirus, Enamovirus, Filoviridae, Flaviviridae, Furovirus, Fuselloviridae, Geminiviridae, Guttaviridae, Hepadnaviridae, Herpesviridae, Hordeivirus, Hypoviridae, Idaeovirus, Inoviridae, Iridoviridae, Leviviridae, Lipothrixviridae, Luteovirus, Machlomovirus, Marafivirus, Microviridae, Myoviridae, Necrovirus, Nodaviridae, Orthomyxoviridae, Papovaviridae, Paramyxoviridae, Partitiviridae, Parvoviridae, Phycodnaviridae, Picornaviridae, Plasmaviridae, Podoviridae, Polydnaviridae, Potexvirus, Potyviridae, Poxviridae, Reoviridae, Retroviridae, Rhabdoviridae, Rhizidiovirus, Sequiviridae, Siphoviridae, Sobemovirus, Tectiviridae, Tenuivirus, Tetraviridae, Tobamovirus, Tobravirus, Togaviridae, Tombusviridae, Totiviridae, Trichovirus, Tymovirus, oder Umbravirus ist, oder ein modifiziertes Kapsomer der genannten Viren und Phagen ist.

9. Verfahren nach einem oder mehreren der vorhergehenden Ansprüche,
**dadurch gekennzeichnet,**
**daß** die Proteinhüllenfragmente monomere Teileinheiten oder Dimere oder Oligomere von Teileinheiten der in Anspruch 6 oder 8 genannten Kapside oder Assemblate sind.

10. Verfahren nach einem oder mehreren der vorhergehenden Ansprüche,
**dadurch gekennzeichnet,**
**daß** das Proteinhüllenfragment in dem ersten Bereich so modifiziert ist, daß dieser erste Bereich eine verbesserte Bindungsaffinität zu der Matrix aufweist, und wahlweise im zweiten Bereich so modifiziert ist, daß dieser zweite Bereich eine verbesserte Bindungsaffinität zu der molekularen Substanz aufweist.

11. Verfahren nach einem oder mehreren der vorhergehenden Ansprüche,
**dadurch gekennzeichnet,**
**daß** der erste Bereich am C-terminalen Teil des Proteinhüllenfragments liegt und der zweite Bereich am N-terminalen Teil, oder der erste Bereich am N-terminalen Teil liegt und der zweite Bereich am N-terminalen Teil , oder die Bereiche in einem anderen Teil des Proteinhüllenfragments, bevorzugt in Loop-Regionen, liegen.

12. Verfahren nach einem oder mehreren der vorhergehenden Ansprüche,
**dadurch gekennzeichnet,**
**daß** das Binden des Proteinhüllenfragments an die Matrix mit dem ersten Bereich reversibel erfolgt.

13. Verfahren nach einem oder mehreren der vorhergehenden Ansprüche,
**dadurch gekennzeichnet,**
**daß** eine irreversible Bindung des Proteinhüllenfragments über den ersten Bereich an die Matrix erfolgt und nach Binden der molekularen Substanz an den zweiten Bereich des Proteinhüllenfragments nur der Teil des Proteinhüllenfragments abgetrennt wird, der den zweiten Bereich des Proteinhüllenfragments umfaßt, und der mit anderen Proteinhüllenfragmenten zu einer Proteinhülle assemblierbar ist.

14. Verfahren nach einem oder mehreren der vorhergehenden Ansprüche,
**dadurch gekennzeichnet,**
**daß** das Proteinhüllenfragment zusätzlich (in einem dritten Bereich) mit einer Markierung versehen ist und/oder die molekulare Substanz mit einer Markierung versehen ist.

15. Verfahren nach Anspruch 14,
**dadurch gekennzeichnet,**
**daß** als Markierung für das Proteinhüllenfragment und/oder die molekulare Substanz ein fluoreszierendes oder radioaktives Molekül, ein spezifisch bindendes Peptidsegment, eine Biotinylierung oder eine andere Markierung verwendet wird.

16. Verfahren nach einem oder mehreren der vorhergehenden Ansprüche,
**dadurch gekennzeichnet,**
**daß** als Matrix eine Chitin-Matrix, Sepharose-Matrix, Dextran-Matrix, oder Diethylaminoethyl-Matrix, verwendet wird.

17. Verfahren nach einem oder mehreren der vorhergehenden Ansprüche,
**dadurch gekennzeichnet,**
**daß** die Matrix in fester Form oder als Gel vorliegt.

18. Verfahren nach einem oder mehreren der vorhergehenden Ansprüche,
**dadurch gekennzeichnet,**
**daß** die Modifikation des ersten Bereichs durch Einfügen einer Inteindomäne in Kombination mit einer Matrixbindungsdomäne, durch spezifische Biotinylierung, oder durch Anhängen eines bindungsvermittelnden Moleküls erfolgt, und/oder daß die Modifikation des zweiten Bereichs durch Fusion mit einem Peptid oder Protein erfolgt, das hohe Affinität zu der zu verpackenden molekularen Substanz besitzt.

19. Matrix mit einem daran gebundenen, assemblierbaren Proteinhüllenfragment, **dadurch gekennzeichnet,**
**daß** an das Proteinhüllenfragment eine in die Proteinhülle in gerichteter Weise einzuschließende molekulare Substanz, nämlich, einzelsträngige oder doppelsträngige DNA, einzelsträngige oder doppelsträngige RNA, Peptide, Peptidhormone, Proteine, Proteindomänen, Glykoproteine, Ribozyme, PNA (Peptide Nucleic Acid), pharmazeutische Wirkstoffe, Nukleotide, Hormone, Lipide, oder Kohlenhydrate oder eine Kombination einer oder mehrerer dieser Substanzen gebunden ist und das Proteinhüllenfragment mit der in gerichteter Weise daran gebundenen molekularen Substanz zumindest teilweise von der Matrix ablösbar ist.

20. Matrix nach Anspruch 19,
**dadurch gekennzeichnet,**
**daß** das Proteinhüllenfragment mit weiteren Proteinhüllenfragmenten assoziiert vorliegt.

## Claims

1. Method for the directed inclusion of molecular substances, namely single-stranded or double-stranded DNA, single-stranded or double-stranded RNA, peptides, peptide hormones, proteins, protein domains, glycoproteins, ribozymes, PNA (Peptide Nucleic Acid), pharmaceutical substances, nucleotides, hormones, lipids, or carbohydrates, or a combination of one or more of these substances, in protein shells with the following steps:
- fixation of a protein shell fragment with a first region to a matrix on which protein shell fragments can be bound;
- bringing the matrix-bound protein shell fragment in contact with the molecular substance, in order to fix the molecular substance to a second region of the protein shell fragment;
- separation of the protein shell fragment with the bound molecular substance, or of one part of the protein shell fragment which contains the bound molecular substance, from the matrix;
and
- assembly of the separated protein shell fragment or a part of it which contains the bound molecular substance, with other protein shell fragments to a protein shell, whereby the separation and the assembly can be done in any order.

2. Method according to claim 1,
**characterized by** the fact,
that the separation and the assembly occur simultaneously, or first the assembly and then the separation occurs.

3. Method according to claim 1 or 2,
**characterized by** the fact,
that after bringing the matrix-bound protein shell fragment in contact with the molecular substance, one or more condensation agents are added, in order to reach a more compact structure of the molecular substances.

4. Method according to any of the preceding claims,
**characterized by** the fact,
that histones, histone-like proteins, polycations, polyarginine, polylysin, spermidine, methylized spermidine, CTAB (cetyltrimethylammoniumbromide), cationic lipids, lipospermine, polyethylene glycol, polyethylene imine, cobalt-amine-compounds or a combination of one or more of these substances are used as condensation substances.

5. Method according to any of the preceding claims,
**characterized by** the fact,
that the molecular substance is DNA in the form of linear or circular plasmids, single-stranded or double-stranded oligonucleotides, chromosomes or chromosome fragments,
or
RNA in the form of antisense-RNA, ribozymes, catalytic RNA, or coding mRNA,
or
that proteins in the form of antibodies, single-chain antibodies, enzymes, structure proteins, or marker proteins are used as molecular substances.

6. Method according to any of the preceding claims,
**characterized by** the fact,
that the protein shell is a virus capsid, a phage capsid, or a macromolecular protein assembly with an inner cavity, preferably a proteasome or a chaperon complex:

7. Method according to any of the preceding claims,
**characterized by** the fact,
that the protein shell consists of identical protein shell fragments or protein shell fragments varying from each other.

8. Method according to any of the preceding claims,
**characterized by** the fact,
that the protein shell fragment is a capsomer of a virus or a phage of Adenoviridae, Arenaviridae, Arterivirus, Astroviridae, Bacilloviridae, Baculoviridae, Badnavirus, Bamaviridae, Birnaviridae, Bromoviridae, Bunyaviridae, Caliciviridae, Capillovirus, Carlavirus, Caulimovirus, Circoviridae, Closterovirus, Comoviridae, Coronaviridae, Corticoviridae, Cystoviridae, Dianthovirus, Enamovirus, Filoviridae, Flaviviridae, Furovirus, Fuselloviridae, Geminiviridae, Guttaviridae, Hepadnaviridae, Herpesviridae, Hordeivirus, Hypoviridae, Idaeovirus, Inoviridae, Iridoviridae, Leviviridae, Lipothrixviridae, Luteovirus, Machlomovirus, Marafivirus, Microviridae, Myoviridae, Necrovirus, Nodaviridae, Orthomyxoviridae, Papovaviridae, Paramyxoviridae, Partitiviridae, Parvoviridae, Phycodnaviridae, Picomaviridae, Plasmaviridae, Podoviridae, Polydnaviridae, Potexvirus, Potyviridae, Poxviridae, Reoviridae, Retroviridae, Rhabdoviridae, Rhizidiovirus, Sequiviridae, Siphoviridae, Sobemovirus, Tectiviridae, Tenuivirus, Tetraviridae, Tobamovirus, Tobravirus, Togaviridae, Tombusviridae, Totiviridae, Trichovirus, Tymovirus, or Umbravirus, or a modified capsomer of the named viruses and phages.

9. Method according to any of the preceding claims,
**characterized by** the fact,
that the protein shell fragments are monomeric subunits or dimers or oligomers of subunits of capsids or assemblies named in claim 6 or 8.

10. Method according to any of the preceding claims,
**characterized by** the fact,
that the protein shell fragment is modified in the first region in such a way that this first region has an improved binding affinity to the matrix, and is optionally modified in the second region in such a way that this second region shows an improved binding affinity to the molecular substance.

11. Method according to any of the preceding claims,
**characterized by** the fact,
that the first region lies at the C-terminal part of the protein shell fragment and the second region at the N-terminal part, or the first region lies at the N-terminal part and the second region at the N-terminal part, or the regions lie in other parts of the protein shell fragment, preferably in loop regions.

12. Method according to any of the preceding claims,
**characterized by** the fact,
that the binding of the protein shell fragment to the matrix with the first region is reversible.

13. Method according to any of the preceding claims,
**characterized by** the fact,
that an irreversible binding of the protein shell fragment to the matrix occurs via the first region and after the binding of the molecular substance to the second region of the protein shell fragment only the part of the protein shell fragment is separated which contains the second region of the protein shell fragment, and which can be assembled with other protein shell fragments to a protein shell.

14. Method according to any of the preceding claims,
**characterized by** the fact,
that the protein shell fragment is supplied additionally (in a third region) with a label and/or the molecular substance is supplied with a label.

15. Method according to claim 14,
**characterized by** the fact, that for the labelling of the protein shell fragment and/or the molecular substance a fluorescent or radioactive molecule, a specifically binding peptide segment, a biotinylation, or another type of labelling is used.

16. Method according to any of the preceding claims,
**characterized by** the fact,
that a chitin matrix, sepharose matrix, dextrane matrix, or diethylaminoethyle matrix is used as the matrix.

17. Method according to any of the preceding claims,
**characterized by** the fact,
that the matrix is in solid form or a gel.

18. Method according to any of the preceding claims,
**characterized by** the fact,
that the modification of the first region occurs by insertion of an intein domain in combination with a matrix binding domain, by specific biotinylation, or by appending of a molecule which mediates binding, and/or that the modification of the second region occurs by fusion with a peptide or protein which has a high affinity for the molecular substances to be packaged.

19. Matrix with a protein shell fragment which is capable of being assembled and is bound to the matrix,
**characterized by** the fact,
that a substance, namely single-stranded or double-stranded DNA, single-stranded or double-stranded RNA, peptides, peptide hormones, proteins, protein domains, glycoproteins, ribozymes, PNA (Peptide Nucleic Acid), pharmaceutical substances, nucleotides, hormones, lipids, or carbohydrates or a combination of one or more of these substances, which is to be enclosed into the protein shell in a directed manner, is bound to the protein shell fragment and that the protein shell fragment with the molecular substance bound to it in a directed manner can be at least partially removed from the matrix.

20. Matrix according to claim 19,
**characterized by** the fact,
that the protein shell fragment is associated with further protein shell fragments.

## Revendications

1. Procédé d'incorporation ciblée de substances moléculaires, à savoir de l'ADN simple brin ou double brin, de l'ARN simple brin ou double brin, des peptides, hormones peptidiques, protéines, domaines protéiques, glycoprotéines, ribozymes, PNA (acide nucléique peptidique), agents actifs pharmaceutiques, nucléotides, hormones, lipides ou hydrates de carbone ou une combinaison d'une ou de plusieurs de ces substances, dans des enveloppes protéiques, comprenant les étapes suivantes :
- liaison d'un fragment d'enveloppe protéique par un premier domaine sur une matrice, sur laquelle des fragments d'enveloppe protéique peuvent être liés ;
- mise en contact du fragment d'enveloppe protéique lié à la matrice avec la substance moléculaire, pour lier la substance moléculaire à un deuxième domaine du fragment d'enveloppe protéique ;
- séparation du fragment d'enveloppe protéique avec la substance moléculaire liée, ou d'une partie du fragment d'enveloppe protéique, qui contient la substance moléculaire liée, de la matrice et
- assemblage du fragment d'enveloppe protéique séparé ou d'une partie de celui-ci, qui contient la substance moléculaire liée, avec d'autres fragments d'enveloppe protéique, en une enveloppe protéique,
la séparation et l'assemblage pouvant être réalisés en ordre quelconque.

2. Procédé selon la revendication 1, **caractérisé en ce que** la séparation et l'assemblage sont réalisés simultanément ou que l'on réalise d'abord l'assemblage, puis la séparation.

3. Procédé selon la revendication 1 ou 2, **caractérisé en ce qu'**après la mise en contact du fragment d'enveloppe protéique lié à la matrice avec la substance moléculaire, une ou plusieurs substances de condensation sont ajoutées pour obtenir une structure plus compacte de la substance moléculaire.

4. Procédé selon l'une ou plusieurs des revendications précédentes, **caractérisé en ce que** comme substance de condensation, on utilise des histones, des protéines de type histone, des polycations, la polyarginine, la polylysine, la spermidine, la spermidine méthylée, le CTAB (bromure de cétyltriméthylammonium), des lipides cationiques, la lipospermine, le polyéthylèneglycol, la polyéthylèneimine, des composés cobalt-amine ou une combinaison d'une ou de plusieurs de ces substances.

5. Procédé selon l'une ou plusieurs des revendications précédentes, **caractérisé en ce que** la substance moléculaire est de l'ADN sous forme de plasmides linéaires ou circulaires, d'oligonucléotides simple brin ou double brin, de chromosomes ou de fragments de chromosome, ou de l'ARN, sous forme d'ARN antisens, de ribozymes, d'ARN catalytique ou d'ARNm codant, ou
l'on met en oeuvre comme substance moléculaire, une protéine sous forme d'anticorps, d'anticorps monocaténaires, d'enzymes, de protéines de structure ou de protéines marqueurs.

6. Procédé selon l'une ou plusieurs des revendications précédentes, **caractérisé en ce que** l'enveloppe protéique est un capside viral, un capside de phage ou un assemblage macromoléculaire de protéines avec espace vide interne, de préférence un protéasome ou un complexe chaperon.

7. Procédé selon l'une ou plusieurs des revendications précédentes, **caractérisé en ce que** l'enveloppe protéique consiste en des fragments d'enveloppe protéique identiques ou différents.

8. Procédé selon l'une ou plusieurs des revendications précédentes, **caractérisé en ce que** le fragment d'enveloppe protéique est un capsomère d'un virus ou d'un phage des Adenoviridae, Arenaviridae, Arterivirus, Astroviridae, Bacilloviridae, Baculoviridae, Badnavirus, Bamaviridae, Birnaviridae, Bromoviridae, Bunyaviridae, Caliciviridae, Capillovirus, Carlavirus, Caulimovirus, Circoviridae, Closteroviridae, Comoviridae, Coronaviridae, Corticoviridae, Cystoviridae, Dianthovirus, Enamovirus, Filoviridae, Flaviviridae, Furovirus, Fuselloviridae, Geminiviridae, Guttaviridae, Hepadnaviridae, Herpesviridae, Hordeivirus, Hypoviridae, Idaeovirus, Inoviridae, Iridoviridae, Leviviridae, Lipothrixviridae, Luteovirus, Machlomovirus, Marafivirus, Microviridae, Myoviridae, Necrovirus, Nodaviridae, Orthomyxoviridae, Papovaviridae, Paramyxoviridae, Partitiviridae, Parvoviridae, Phycodnaviridae, Piocornaviridae, Plasmaviridae, Podoviridae, Polydnaviridae, Potexvirus, Potyviridae, Poxviridae, Reoviridae, Retroviridae, Rhabdoviridae, Rhizidiovirus, Sesquiviridae, Siphoviridae, Sobemovirus, Tectiviridae, Tenuivirus, Tetraviridae, Tobamovirus, Tobravirus, Togaviridae, Tombusviridae, Totiviridae, Trichovirus, Tymovirus ou Umbravirus, ou un capsomère modifié des virus et phages indiqués.

9. Procédé selon l'une ou plusieurs des revendications précédentes, **caractérisé en ce que** les fragments d'enveloppe protéique sont des unités partielles monomères ou des dimères ou oligomères d'unités partielles des capsides ou assemblages indiqués à la revendication 6 ou 8.

10. Procédé selon l'une ou plusieurs des revendications précédentes, **caractérisé en ce que** le fragment d'enveloppe protéique est modifié dans le premier domaine, de sorte que ce premier domaine présente une affinité de liaison améliorée pour la matrice, et est modifiée dans le deuxième domaine, si nécessaire, de sorte que ce deuxième domaine présente une affinité de liaison améliorée pour la substance moléculaire.

11. Procédé selon l'une ou plusieurs des revendications précédentes, **caractérisé en ce que** le premier domaine se trouve sur la partie C-terminale du fragment d'enveloppe protéique et le deuxième domaine sur la partie N-terminale, ou le premier domaine se trouve sur la partie N-terminale du fragment d'enveloppe protéique et le deuxième domaine sur la partie N-terminale, ou les domaines se trouvent dans une autre partie du fragment d'enveloppe protéique, de préférence dans les régions de boucle.

12. Procédé selon l'une ou plusieurs des revendications précédentes, **caractérisé en ce que** la liaison du fragment d'enveloppe protéique sur la matrice est réalisée de manière réversible, par le premier domaine.

13. Procédé selon l'une ou plusieurs des revendications précédentes, **caractérisé en ce qu'**une liaison irréversible du fragment d'enveloppe protéique est réalisée par le premier domaine sur la matrice et après liaison de la substance moléculaire sur le deuxième domaine du fragment d'enveloppe protéique, seule une partie du fragment d'enveloppe protéique est séparée, laquelle comprend le deuxième domaine du fragment d'enveloppe protéique, et laquelle peut être assemblée à d'autres fragments d'enveloppe protéique en une enveloppe protéique.

14. Procédé selon l'une ou plusieurs des revendications précédentes, **caractérisé en ce que** le fragment d'enveloppe protéique est muni en outre (dans un troisième domaine), d'un marquage et/ou la substance moléculaire est munie d'un marquage.

15. Procédé selon la revendication 14, **caractérisé en ce que** comme marquage pour le fragment d'enveloppe protéique et/ou de la substance moléculaire, on utilise une molécule fluorescente ou radioactive, un segment peptidique se liant spécifiquement, une biotinylation ou un autre marquage.

16. Procédé selon l'une ou plusieurs des revendications précédentes, **caractérisé en ce que** comme matrice, on utilise une matrice chitine, une matrice sépharose, une matrice dextran ou une matrice diéthylaminoéthyle.

17. Procédé selon l'une ou plusieurs des revendications précédentes, **caractérisé en ce que** la matrice se présente sous forme solide ou comme gel.

18. Procédé selon l'une ou plusieurs des revendications précédentes, **caractérisé en ce que** la modification du premier domaine est réalisée par insertion d'un domaine d'intéine en combinaison avec un domaine de liaison de matrice, par biotinylation spécifique ou par fixation d'une molécule intermédiaire de liaison, et/ou **en ce que** la modification du deuxième domaine est réalisée par fusion avec un peptide ou une protéine, qui possède une affinité élevée pour la substance moléculaire à incorporer.

19. Matrice avec un fragment d'enveloppe protéique assemblable lié à celle-ci, **caractérisée en ce que** sur le fragment d'enveloppe protéique est liée une substance moléculaire à incorporer de manière ciblée dans l'enveloppe protéique, à savoir de l'ADN simple brin ou double brin, de l'ARN simple brin ou double brin, des peptides, hormones peptidiques, protéines, domaines protéiques, glycoprotéines, ribozymes, PNA (acide nucléique peptidique), agents actif pharmaceutiques, nucléotides, hormones, lipides ou hydrates de carbone ou une combinaison d'une ou de plusieurs de ces substances, et **en ce que** le fragment d'enveloppe protéique avec la substance moléculaire qui lui est liée de manière ciblée peut être au moins partiellement séparé de la matrice.

20. Matrice selon la revendication 19, **caractérisée en ce que** le fragment d'enveloppe protéique est présent sous forme associée à d'autres fragments d'enveloppe protéique.


### SEQUENZPROTOKOLL

<110> ACGT ProGenomics AG
<120> Verfahren zur gerichteten Verpackung von molekularen Substanzen in Proteinhüllen
<130> P12602
<140>
   <141>
<150> DE - 199 52 982.5
   <151> 1999-11-03
<160> 10
<170> PatentIn Ver. 2.1
<210> 1
   <211> 1152
   <212> DNA
   <213> Künstliche Sequenz
<220>
   <221> CDS
   <222> (1)..(1152)
<220>
   <223> Beschreibung der künstlichen Sequenz: Polyomavirus VP1-Gen mit Austausch aller Cysteine gegen Serin, und Austausch von Threonin 249 gegen Cystein (PyVP1-CallS-T249C)
<300>
<400> 1
   atg gcc ccc aaa aga aaa agc ggc gtc tct aaa agc gag aca aaa agc    48
   Met Ala Pro Lys Arg Lys Ser Gly Val Ser Lys Ser Glu Thr Lys Ser
    1               5                  10                  15
   aca aag gct agc cca aga ccc gca ccc gtt ccc aaa ctg ctt att aaa    96
   Thr Lys Ala Ser Pro Arg Pro Ala Pro Val Pro Lys Leu Leu Ile Lys
               20                  25                  30
   ggg ggt atg gag gtg ctg gac ctt gtg aca ggg cca gac agt gtg aca   144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct    192
   Glu Ile Glu Ala Phe Leu Asn Pro Arg Met Gly Gln Pro Pro Thr Pro
        50                  55                  60
   gaa agc cta aca gag gga ggg caa tac tat ggt tgg agc aga ggg att    240
   Glu Ser Leu Thr Glu Gly Gly Gln Tyr Tyr Gly Trp Ser Arg Gly Ile
    65                  70                  75                  80
   aat ttg gct aca tca gat aca gag gat tcc cca gga aat aat aca ctt    288
   Asn Leu Ala Thr Ser Asp Thr Glu Asp Ser Pro Gly Asn Asn Thr Leu
                    85                  90                  95
   ccc aca tgg agt atg gca aag ctc cag ctt ccc atg ctc aat gag gac    336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc    384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc    480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag    528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta    576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc    768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc    912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat    960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
       370                 375                 380
<210> 2
   <211> 384
   <212> PRT
   <213> Künstliche Sequenz
   <223> Beschreibung der künstlichen Sequenz: Polyomavirus VP1-Gen mit Austausch aller Cysteine gegen Serin, und Austausch von Threonin 249 gegen Cystein (PyVP1-CallS-T249C)
<400> 2
   Met Ala Pro Lys Arg Lys Ser Gly Val Ser Lys Ser Glu Thr Lys Ser
   1               5                   10                  15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Ser Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Val Asn Thr Lys Gly Ile Ser Thr Pro Val Glu Gly
   145                 150                 155                 160
   Ser Gln Tyr His Val Phe Ala Val Gly Gly Glu Pro Leu Asp Leu Gln
                   165                 170                 175
   Gly Leu Val Thr Asp Ala Arg Thr Lys Tyr Lys Glu Glu Gly Val Val
               180                 185                 190
   Thr Ile Lys Thr Ile Thr Lys Lys Asp Met Val Asn Lys Asp Gln Val
           195                 200                 205
   Leu Asn Pro Ile Ser Lys Ala Lys Leu Asp Lys Asp Gly Met Tyr Pro
       210                 215                 220
   Val Glu Ile Trp His Pro Asp Pro Ala Lys Asn Glu Asn Thr Arg Tyr
   225                 230                 235                 240
   Phe Gly Asn Tyr Thr Gly Gly Thr Cys Thr Pro Pro Val Leu Gln Phe
                   245                 250                 255
   Thr Asn Thr Leu Thr Thr Val Leu Leu Asp Glu Asn Gly Val Gly Pro
               260                 265                 270
   Leu Ser Lys Gly Glu Gly Leu Tyr Leu Ser Ser Val Asp Ile Met Gly
           275                 280                 285
   Trp Arg Val Thr Arg Asn Tyr Asp Val His His Trp Arg Gly Leu Pro
       290                 295                 300
   Arg Tyr Phe Lys Ile Thr Leu Arg Lys Arg Trp Val Lys Asn Pro Tyr
   305                 310                 315                 320
   Pro Met Ala Ser Leu Ile Ser Ser Leu Phe Asn Asn Met Leu Pro Gln
                   325                 330                 335
   Val Gln Gly Gln Pro Met Glu Gly Glu Asn Thr Gln Val Glu Glu Val
               340                 345                 350
   Arg Val Tyr Asp Gly Thr Glu Pro Val Pro Gly Asp Pro Asp Met Thr
           355                 360                 365
   Arg Tyr Val Asp Arg Phe Gly Lys Thr Lys Thr Val Phe Pro Pro Gly
       370                 375                 380
<210> 3
   <211> 1176
   <212> DNA
   <213> Künstliche Sequenz
<220>
   <223> Beschreibung der künstlichen Sequenz: Fusion aus modifiziertem Polyomavirus VP1-Gen (PyVVP1-CallS-T249C) am 5'-Ende mit protaminähnlicher RNA-Bindungsdomäne aus Hepatitis-B-Virus p21.5 (111-PyVP1)
<220>
   <221> misc_feature
   <222> (4) .. (78)
   <223> RNA-Bindungsmotiv aus dem Protein p21.5 von Hepatitis-B-Virus
<220>
   <221> CDS
   <222> (1)..(1176)
<400> 3
   atg gcg cgt cgt cgt gat cgt ggc cgt agc cgt cgt cgt gat cgt ggt    48
   Met Ala Arg Arg Arg Asp Arg Gly Arg Ser Arg Arg Arg Asp Arg Gly
    1               5                  10                  15
   cgt agc cgt cgt cgt gat cgt ggt cgt agc gct agc cca aga ccc gca    96
   Arg Ser Arg Arg Arg Asp Arg Gly Arg Ser Ala Ser Pro Arg Pro Ala
               20                  25                  30
   ccc gtt ccc aaa ctg ctt att aaa ggg ggt atg gag gtg ctg gac ctt   144
   Pro Val Pro Lys Leu Leu Ile Lys Gly Gly Met Glu Val Leu Asp Leu
           35                  40                  45
   gtg aca ggg cca gac agt gtg aca gaa ata gaa gct ttt ctg aac ccc   192
   Val Thr Gly Pro Asp Ser Val Thr Glu Ile Glu Ala Phe Leu Asn Pro
       50                  55                  60
   aga atg ggg cag cca ccc acc cct gaa agc cta aca gag gga ggg caa   240
   Arg Met Gly Gln Pro Pro Thr Pro Glu Ser Leu Thr Glu Gly Gly Gln
   65                  70                  75                  80
   tac tat ggt tgg agc aga ggg att aat ttg gct aca tca gat aca gag   288
   Tyr Tyr Gly Trp Ser Arg Gly Ile Asn Leu Ala Thr Ser Asp Thr Glu
                   85                  90                  95
   gat tcc cca gga aat aat aca ctt ccc aca tgg agt atg gca aag ctc   336
   Asp Ser Pro Gly Asn Asn Thr Leu Pro Thr Trp Ser Met Ala Lys Leu
               100                 105                 110
   cag ctt ccc atg ctc aat gag gac ctc acg tct gac acc cta caa atg   384
   Gln Leu Pro Met Leu Asn Glu Asp Leu Thr Ser Asp Thr Leu Gln Met
           115                 120                 125
   tgg gag gca gtc tca gtg aaa acc gag gtg gtg ggc tct ggc tca ctg   432
   Trp Glu Ala Val Ser Val Lys Thr Glu Val Val Gly Ser Gly Ser Leu
       130                 135                 140
   tta gat gtg cat ggg ttc aac aaa ccc aca gat aca gta aac aca aaa   480
   Leu Asp Val His Gly Phe Asn Lys Pro Thr Asp Thr Val Asn Thr Lys
   145                 150                 155                 160
   gga att tcc act cca gtg gaa ggc agc caa tat cat gtg ttt gct gtg   528
   Gly Ile Ser Thr Pro Val Glu Gly Ser Gln Tyr His Val Phe Ala Val
                   165                 170                 175
   ggc ggg gaa ccg ctt gac ctc cag gga ctt gtg aca gat gcc aga aca   576
   Gly Gly Glu Pro Leu Asp Leu Gln Gly Leu Val Thr Asp Ala Arg Thr
               180                 185                 190
   aaa tac aag gaa gaa ggg gta gta aca atc aaa aca atc aca aag aag     624
   Lys Tyr Lys Glu Glu Gly Val Val Thr Ile Lys Thr Ile Thr Lys Lys
           195                 200                 205
   gac atg gtc aac aaa gac caa gtc ctg aat cca att agc aag gcc aag     672
   Asp Met Val Asn Lys Asp Gln Val Leu Asn Pro Ile Ser Lys Ala Lys
       210                 215                 220
   ctg gat aag gac gga atg tat cca gtt gaa atc tgg cat cca gat cca     720
   Leu Asp Lys Asp Gly Met Tyr Pro Val Glu Ile Trp His Pro Asp Pro
   225                 230                 235                 240
   gca aaa aat gag aac aca agg tac ttt ggc aat tac act gga ggc acg     768
   Ala Lys Asn Glu Asn Thr Arg Tyr Phe Gly Asn Tyr Thr Gly Gly Thr
                   245                 250                 255
   tgc acc cca ccc gtc ctg cag ttc aca aac acc ctg aca act gtg ctc     816
   Cys Thr Pro Pro Val Leu Gln Phe Thr Asn Thr Leu Thr Thr Val Leu
               260                 265                 270
   cta gat gaa aat gga gtt ggg ccc ctc agc aaa gga gaa ggt cta tac     864
   Leu Asp Glu Asn Gly Val Gly Pro Leu Ser Lys Gly Glu Gly Leu Tyr
           275                 280                 285
   ctc tcg agc gta gat ata atg ggc tgg aga gtt aca aga aac tat gat     912
   Leu Ser Ser Val Asp Ile Met Gly Trp Arg Val Thr Arg Asn Tyr Asp
       290                 295                 300
   gtc cat cac tgg aga ggg ctt ccc aga tat ttc aaa atc acc ctg aga     960
   Val His His Trp Arg Gly Leu Pro Arg Tyr Phe Lys Ile Thr Leu Arg
   305                 310                 315                 320
   aaa aga tgg gtc aaa aat ccc tat ccc atg gcc tcc ctc ata agt tcc    1008
   Lys Arg Trp Val Lys Asn Pro Tyr Pro Met Ala Ser Leu Ile Ser Ser
                   325                 330                 335
   ctt ttc aac aac atg ctc ccc caa gtg cag ggc caa ccc atg gaa ggg    1056
   Leu Phe Asn Asn Met Leu Pro Gln Val Gln Gly Gln Pro Met Glu Gly
               340                 345                 350
   gag aac acc cag gta gag gag gtt aga gtg tat gat ggg act gaa cct    1104
   Glu Asn Thr Gln Val Glu Glu Val Arg Val Tyr Asp Gly Thr Glu Pro
           355                 360                 365
   gta ccg ggg gac cct gat atg acg cgc tat gtt gac cgc ttt gga aaa    1152
   Val Pro Gly Asp Pro Asp Met Thr Arg Tyr Val Asp Arg Phe Gly Lys
       370                 375                 380
   aca aag act gta ttt cct ccc ggg                                    1176
   Thr Lys Thr Val Phe Pro Pro Gly
   385                 390
<210> 4
   <211> 392
   <212> PRT
   <213> Künstliche Sequenz
   <223> Beschreibung der künstlichen Sequenz: Fusion aus modifiziertem Polyomavirus VP1-Gen (PyVVP1-Calls-T249C) am 5'-Ende mit protaminähnlicher RNA-Bindungsdomäne aus Hepatitis-B-Virus p21.5 (111-PyVP1)
<400> 4
   Met Ala Arg Arg Arg Asp Arg Gly Arg Ser Arg Arg Arg Asp Arg Gly
    1               5                  10                  15
   Arg Ser Arg Arg Arg Asp Arg Gly Arg Ser Ala Ser Pro Arg Pro Ala
               20                  25                  30
   Pro Val Pro Lys Leu Leu Ile Lys Gly Gly Met Glu Val Leu Asp Leu
           35                  40                  45
   Val Thr Gly Pro Asp Ser Val Thr Glu Ile Glu Ala Phe Leu Asn Pro
       50                  55                  60
   Arg Met Gly Gln Pro Pro Thr Pro Glu Ser Leu Thr Glu Gly Gly Gln
   65                  70                  75                  80
   Tyr Tyr Gly Trp Ser Arg Gly Ile Asn Leu Ala Thr Ser Asp Thr Glu
                   85                  90                  95
   Asp Ser Pro Gly Asn Asn Thr Leu Pro Thr Trp Ser Met Ala Lys Leu
               100                 105                 110
   Gln Leu Pro Met Leu Asn Glu Asp Leu Thr Ser Asp Thr Leu Gln Met
           115                 120                 125
   Trp Glu Ala Val Ser Val Lys Thr Glu Val Val Gly Ser Gly Ser Leu
       130                 135                 140
   Leu Asp Val His Gly Phe Asn Lys Pro Thr Asp Thr Val Asn Thr Lys
   145                 150                 155                 160
   Gly Ile Ser Thr Pro Val Glu Gly Ser Gln Tyr His Val Phe Ala Val
   165 170 175
   Gly Gly Glu Pro Leu Asp Leu Gln Gly Leu Val Thr Asp Ala Arg Thr
   180 185 190
   Lys Tyr Lys Glu Glu Gly Val Val Thr Ile Lys Thr Ile Thr Lys Lys
   195 200 205
   Asp Met Val Asn Lys Asp Gln Val Leu Asn Pro Ile Ser Lys Ala Lys
   210 215 220
   Leu Asp Lys Asp Gly Met Tyr Pro Val Glu Ile Trp His Pro Asp Pro
   225 230 235 240
   Ala Lys Asn Glu Asn Thr Arg Tyr Phe Gly Asn Tyr Thr Gly Gly Thr
   245 250 255
   Cys Thr Pro Pro Val Leu Gln Phe Thr Asn Thr Leu Thr Thr Val Leu
   260 265 270
   Leu Asp Glu Asn Gly Val Gly Pro Leu Ser Lys Gly Glu Gly Leu Tyr
   275 280 285
   Leu Ser Ser Val Asp Ile Met Gly Trp Arg Val Thr Arg Asn Tyr Asp
   290 295 300
   Val His His Trp Arg Gly Leu Pro Arg Tyr Phe Lys Ile Thr Leu Arg
   305 310 315 320
   Lys Arg Trp Val Lys Asn Pro Tyr Pro Met Ala Ser Leu Ile Ser Ser
   325 330 335
   Leu Phe Asn Asn Met Leu Pro Gln Val Gln Gly Gln Pro Met Glu Gly
   340 345 350
   Glu Asn Thr Gln Val Glu Glu Val Arg Val Tyr Asp Gly Thr Glu Pro
   355 360 365
   Val Pro Gly Asp Pro Asp Met Thr Arg Tyr Val Asp Arg Phe Gly Lys
   370 375 380
   Thr Lys Thr Val Phe Pro Pro Gly
   385 390
<210> 5
   <211> 1176
   <212> DNA
   <213> Künstliche Sequenz
<220>
   <223> Beschreibung der künstlichen Sequenz: Modifiziertes Polyomavirus VP1-Gen (PyVP1-CallS-T249C) mit am 5'-Ende anfusionierter protaminähnlicher DNA-Bindungsdomäne aus Hepatitis-B-Virus p21.5 (234-PyVP1)
<220>
   <221> CDS
   <222> (1)..(1176)
<220>
   <221> misc_feature
   <222> (4)..(78)
   <223> DNA-bindende Domäne aus dem Protein p21.5 des Hepatitis-B-Virus
<400> 5
   atg gcc agc ccg cgt cgt cgt acc cca agc cca cgt cgt cgt cgt agc     48
   Met Ala Ser Pro Arg Arg Arg Thr Pro Ser Pro Arg Arg Arg Arg Ser
    1               5                  10                  15
   cag agc ccg cgg tcg tcc ggt cgt agc cag gct agc cca aga ccc gca     96
   Gln Ser Pro Arg Ser Ser Gly Arg Ser Gln Ala Ser Pro Arg Pro Ala
               20                  25                  30
   ccc gtt ccc aaa ctg ctt att aaa ggg ggt atg gag gtg ctg gac ctt    144
   Pro Val Pro Lys Leu Leu Ile Lys Gly Gly Met Glu Val Leu Asp Leu
           35                  40                  45
   gtg aca ggg cca gac agt gtg aca gaa ata gaa gct ttt ctg aac ccc    192
   Val Thr Gly Pro Asp Ser Val Thr Glu Ile Glu Ala Phe Leu Asn Pro
       50                  55                  60
   aga atg ggg cag cca ccc acc cct gaa agc cta aca gag gga ggg caa    240
   Arg Met Gly Gln Pro Pro Thr Pro Glu Ser Leu Thr Glu Gly Gly Gln
   65                  70                  75                  80
   tac tat ggt tgg agc aga ggg att aat ttg gct aca tca gat aca gag    288
   Tyr Tyr Gly Trp Ser Arg Gly Ile Asn Leu Ala Thr Ser Asp Thr Glu
                   85                  90                  95
   gat tcc cca gga aat aat aca ctt ccc aca tgg agt atg gca aag ctc    336
   Asp Ser Pro Gly Asn Asn Thr Leu Pro Thr Trp Ser Met Ala Lys Leu
               100                 105                 110
   cag ctt ccc atg ctc aat gag gac ctc acg tct gac acc cta caa atg    384
   Gln Leu Pro Met Leu Asn Glu Asp Leu Thr Ser Asp Thr Leu Gln Met
           115                 120                 125
   tgg gag gca gtc tca gtg aaa acc gag gtg gtg ggc tct ggc tca ctg    432
   Trp Glu Ala Val Ser Val Lys Thr Glu Val Val Gly Ser Gly Ser Leu
       130                 135                 140
   tta gat gtg cat ggg ttc aac aaa ccc aca gat aca gta aac aca aaa    480
   Leu Asp Val His Gly Phe Asn Lys Pro Thr Asp Thr Val Asn Thr Lys
   145                 150                 155                 160
   gga att tcc act cca gtg gaa ggc agc caa tat cat gtg ttt gct gtg    528
   Gly Ile Ser Thr Pro Val Glu Gly Ser Gln Tyr His Val Phe Ala Val
                   165                 170                 175
   ggc ggg gaa ccg ctt gac ctc cag gga ctt gtg aca gat gcc aga aca    576
   Gly Gly Glu Pro Leu Asp Leu Gln Gly Leu Val Thr Asp Ala Arg Thr
               180                 185                 190
   aaa tac aag gaa gaa ggg gta gta aca atc aaa aca atc aca aag aag    624
   Lys Tyr Lys Glu Glu Gly Val Val Thr Ile Lys Thr Ile Thr Lys Lys
           195                 200                 205
   gac atg gtc aac aaa gac caa gtc ctg aat cca att agc aag gcc aag    672
   Asp Met Val Asn Lys Asp Gln Val Leu Asn Pro Ile Ser Lys Ala Lys
       210                 215                 220
   ctg gat aag gac gga atg tat cca gtt gaa atc tgg cat cca gat cca    720
   Leu Asp Lys Asp Gly Met Tyr Pro Val Glu Ile Trp His Pro Asp Pro
   225                 230                 235                 240
   gca aaa aat gag aac aca agg tac ttt ggc aat tac act gga ggc acg    768
   Ala Lys Asn Glu Asn Thr Arg Tyr Phe Gly Asn Tyr Thr Gly Gly Thr
                   245                 250                 255
   tgc acc cca ccc gtc ctg cag ttc aca aac acc ctg aca act gtg ctc    816
   Cys Thr Pro Pro Val Leu Gln Phe Thr Asn Thr Leu Thr Thr Val Leu
               260                 265                 270
   cta gat gaa aat gga gtt ggg ccc ctc agc aaa gga gaa ggt cta tac    864
   Leu Asp Glu Asn Gly Val Gly Pro Leu Ser Lys Gly Glu Gly Leu Tyr
           275                 280                 285
   ctc tcg agc gta gat ata atg ggc tgg aga gtt aca aga aac tat gat    912
   Leu Ser Ser Val Asp Ile Met Gly Trp Arg Val Thr Arg Asn Tyr Asp
       290                 295                 300
   gtc cat cac tgg aga ggg ctt ccc aga tat ttc aaa atc acc ctg aga     960
   Val His His Trp Arg Gly Leu Pro Arg Tyr Phe Lys Ile Thr Leu Arg
   305                 310                 315                 320
   aaa aga tgg gtc aaa aat ccc tat ccc atg gcc tcc ctc ata agt tcc    1008
   Lys Arg Trp Val Lys Asn Pro Tyr Pro Met Ala Ser Leu Ile Ser Ser
                   325                 330                 335
   ctt ttc aac aac atg ctc ccc caa gtg cag ggc caa ccc atg gaa ggg    1056
   Leu Phe Asn Asn Met Leu Pro Gln Val Gln Gly Gln Pro Met Glu Gly
               340                 345                 350
   gag aac acc cag gta gag gag gtt aga gtg tat gat ggg act gaa cct    1104
   Glu Asn Thr Gln Val Glu Glu Val Arg Val Tyr Asp Gly Thr Glu Pro
           355                 360                 365
   gta ccg ggg gac cct gat atg acg cgc tat gtt gac cgc ttt gga aaa    1152
   Val Pro Gly Asp Pro Asp Met Thr Arg Tyr Val Asp Arg Phe Gly Lys
       370                 375                 380
   aca aag act gta ttt cct ccc ggg                                    1176
   Thr Lys Thr Val Phe Pro Pro Gly
   385                 390
<210> 6
   <211> 392
   <212> PRT
   <213> Künstliche Sequenz
   <223> Beschreibung der künstlichen Sequenz: Modifiziertes Polyomavirus VP1-Gen (PyVP1-CallS-T249C) mit am 5'-Ende anfusionierter protaminähnlicher DNA-Bindungsdomäne aus Hepatitis-B-Virus p21.5 (234-PyVP1)
<400> 6
   Met Ala Ser Pro Arg Arg Arg Thr Pro Ser Pro Arg Arg Arg Arg Ser
   1               5                   10                  15
   Gln Ser Pro Arg Ser Ser Gly Arg Ser Gln Ala Ser Pro Arg Pro Ala
               20                  25                  30
   Pro Val Pro Lys Leu Leu Ile Lys Gly Gly Met Glu Val Leu Asp Leu
           35                  40                  45
   Val Thr Gly Pro Asp Ser Val Thr Glu Ile Glu Ala Phe Leu Asn Pro
       50                  55                  60
   Arg Met Gly Gln Pro Pro Thr Pro Glu Ser Leu Thr Glu Gly Gly Gln
   65 70 75 80
   Tyr Tyr Gly Trp Ser Arg Gly Ile Asn Leu Ala Thr Ser Asp Thr Glu
   85 90 95
   Asp Ser Pro Gly Asn Asn Thr Leu Pro Thr Trp Ser Met Ala Lys Leu
   100 105 110
   Gln Leu Pro Met Leu Asn Glu Asp Leu Thr Ser Asp Thr Leu Gln Met
   115 120 125
   Trp Glu Ala Val Ser Val Lys Thr Glu Val Val Gly Ser Gly Ser Leu
   130 135 140
   Leu Asp Val His Gly Phe Asn Lys Pro Thr Asp Thr Val Asn Thr Lys
   145 150 155 160
   Gly Ile Ser Thr Pro Val Glu Gly Ser Gln Tyr His Val Phe Ala Val
   165 170 175
   Gly Gly Glu Pro Leu Asp Leu Gln Gly Leu Val Thr Asp Ala Arg Thr
   180 185 190
   Lys Tyr Lys Glu Glu Gly Val Val Thr Ile Lys Thr Ile Thr Lys Lys
   195 200 205
   Asp Met Val Asn Lys Asp Gln Val Leu Asn Pro Ile Ser Lys Ala Lys
   210 215 220
   Leu Asp Lys Asp Gly Met Tyr Pro Val Glu Ile Trp His Pro Asp Pro
   225 230 235 240
   Ala Lys Asn Glu Asn Thr Arg Tyr Phe Gly Asn Tyr Thr Gly Gly Thr
   245 250 255
   Cys Thr Pro Pro Val Leu Gln Phe Thr Asn Thr Leu Thr Thr Val Leu
   260 265 270
   Leu Asp Glu Asn Gly Val Gly Pro Leu Ser Lys Gly Glu Gly Leu Tyr
   275 280 285
   Leu Ser Ser Val Asp Ile Met Gly Trp Arg Val Thr Arg Asn Tyr Asp
   290 295 300
   Val His His Trp Arg Gly Leu Pro Arg Tyr Phe Lys Ile Thr Leu Arg
   305 310 315 320
   Lys Arg Trp Val Lys Asn Pro Tyr Pro Met Ala Ser Leu Ile Ser Ser
   325 330 335
   Leu Phe Asn Asn Met Leu Pro Gln Val Gln Gly Gln Pro Met Glu Gly
   340 345 350
   Glu Asn Thr Gln Val Glu Glu Val Arg Val Tyr Asp Gly Thr Glu Pro
   355 360 365
   Val Pro Gly Asp Pro Asp Met Thr Arg Tyr Val Asp Arg Phe Gly Lys
   370 375 380
   Thr Lys Thr Val Phe Pro Pro Gly
   385 390
<210> 7
   <211> 765
   <212> DNA
   <213> Künstliche Sequenz
<220>
   <223> Beschreibung der künstlichen Sequenz: Enhanced GFP-Protein, das C-terminal eine zusätzliche prolinreiche Sequenz anfusioniert hat (GFP-PPLP)
<220>
   <221> CDS
   <222> (1)..(765)
<220>
   <221> misc_feature
   <222> (736)..(765)
   <223> inseriertes Polyprolin-Segment
<400> 7
   atg gtg agc aag ggc gag gag ctg ttc acc ggg gtg gtg ccc atc ctg 48
   Met Val Ser Lys Gly Glu Glu Leu Phe Thr Gly Val Val Pro Ile Leu
   1 5 10 15
   gtc gag ctg gac ggc gac gta aac ggc cac aag ttc agc gtg tcc ggc 96
   Val Glu Leu Asp Gly Asp Val Asn Gly His Lys Phe Ser Val Ser Gly
   20 25 30
   gag ggc gag ggc gat gcc acc tac ggc aag ctg acc ctg aag ttc atc 144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc     192
   Cys Thr Thr Gly Lys Leu Pro Val Pro Trp Pro Thr Leu Val Thr Thr
       50                  55                  60
   ctg acc tac ggc gtg cag tgc ttc agc cgc tac ccc gac cac atg aag     240
   Leu Thr Tyr Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag     288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag     336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc     384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac     480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc     528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg     624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac tta agc     720
   Val Thr Ala Ala Gly Ile Thr Leu Gly Met Asp Glu Leu Tyr Leu Ser
   225                 230                 235                 240
   cga cgt gcc tca ggt ccg ccg cct cca ccg cca ccg cct tta ccc          765
   Arg Arg Ala Ser Gly Pro Pro Pro Pro Pro Pro Pro Pro Leu Pro
                   245                 250                 255
<210> 8
   <211> 255
   <212> PRT
   <213> Künstliche Sequenz
   <223> Beschreibung der künstlichen Sequenz: Enhanced GFP-Protein, das C-terminal eine zusätzliche prolinreiche Sequenz anfusioniert hat (GFP-PPLP)
<400> 8
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Leu Ser
   225                 230                 235                 240
   Arg Arg Ala Ser Gly Pro Pro Pro Pro Pro Pro Pro Pro Leu Pro
                   245                 250                 255
<210> 9
   <211> 1251
   <212> DNA
   <213> Künstliche Sequenz
<220>
   <223> Beschreibung der künstlichen Sequenz: WW-Domäne N-terminal an Polyomavirus VP1, das 4 der 6 Cysteine durch Serin ersetzt hat und den Austausch VP1-Thr 249 nach Cys hat (PyVP1-WW1)
<220>
   <221> misc_feature
   <222> (10)..(93)
   <223> Inserierte WW-Domäne
<220>
   <221> CDS
   <222> (1)..(1251)
<400> 9
   atg agc ggc tgg aca gaa cat aaa tca cct gat gga agg act tat tat     48
   Met Ser Gly Trp Thr Glu His Lys Ser Pro Asp Gly Arg Thr Tyr Tyr
    1               5                  10                  15
   tac aat act gaa aca aaa cag tct acc tgg gaa aag cca gat gat gga     96
   Tyr Asn Thr Glu Thr Lys Gln Ser Thr Trp Glu Lys Pro Asp Asp Gly
               20                  25                  30
   cat atg gcc ccc aaa aga aaa agc ggc gtc tct aaa tct gag aca aaa    144
   His Met Ala Pro Lys Arg Lys Ser Gly Val Ser Lys Ser Glu Thr Lys
           35                  40                  45
   agc aca aag gcc tgt cca aga ccc gca ccc gtt ccc aaa ctg ctt att    192
   Ser Thr Lys Ala Cys Pro Arg Pro Ala Pro Val Pro Lys Leu Leu Ile
       50                  55                  60
   aaa ggg ggt atg gag gtg ctg gac ctt gtg aca ggg cca gac agt gtg    240
   Lys Gly Gly Met Glu Val Leu Asp Leu Val Thr Gly Pro Asp Ser Val
   65                  70                  75                  80
   aca gaa ata gaa gct ttt ctg aac ccc aga atg ggg cag cca ccc acc    288
   Thr Glu Ile Glu Ala Phe Leu Asn Pro Arg Met Gly Gln Pro Pro Thr
                   85                  90                  95
   cct gaa agc cta aca gag gga ggg caa tac tat ggt tgg agc aga ggg    336
   Pro Glu Ser Leu Thr Glu Gly Gly Gln Tyr Tyr Gly Trp Ser Arg Gly
               100                 105                 110
   att aat ttg gct aca tca gat aca gag gat tcc cca gga aat aat aca    384
   Ile Asn Leu Ala Thr Ser Asp Thr Glu Asp Ser Pro Gly Asn Asn Thr
           115                 120                 125
   ctt ccc aca tgg agt atg gca aag ctc cag ctt ccc atg ctc aat gag    432
   Leu Pro Thr Trp Ser Met Ala Lys Leu Gln Leu Pro Met Leu Asn Glu
       130                 135                 140
   gac ctc acc tgt gac acc cta caa atg tgg gag gca gtc tca gtg aaa    480
   Asp Leu Thr Cys Asp Thr Leu Gln Met Trp Glu Ala Val Ser Val Lys
   145                 150                 155                 160
   acc gag gtg gtg ggc tct ggc tca ctg tta gat gtg cat ggg ttc aac    528
   Thr Glu Val Val Gly Ser Gly Ser Leu Leu Asp Val His Gly Phe Asn
                   165                 170                 175
   aaa ccc aca gat aca gta aac aca aaa gga att tcc act cca gtg gaa    576
   Lys Pro Thr Asp Thr Val Asn Thr Lys Gly Ile Ser Thr Pro Val Glu
               180                 185                 190
   ggc agc caa tat cat gtg ttt gct gtg ggc ggg gaa ccg ctt gac ctc    624
   Gly Ser Gln Tyr His Val Phe Ala Val Gly Gly Glu Pro Leu Asp Leu
           195                 200                 205
   cag gga ctt gtg aca gat gcc aga aca aaa tac aag gaa gaa ggg gta    672
   Gln Gly Leu Val Thr Asp Ala Arg Thr Lys Tyr Lys Glu Glu Gly Val
       210                 215                 220
   gta aca atc aaa aca atc aca aag aag gac atg gtc aac aaa gac caa    720
   Val Thr Ile Lys Thr Ile Thr Lys Lys Asp Met Val Asn Lys Asp Gln
   225                 230                 235                 240
   gtc ctg aat cca att agc aag gcc aag ctg gat aag gac gga atg tat     768
   Val Leu Asn Pro Ile Ser Lys Ala Lys Leu Asp Lys Asp Gly Met Tyr
                   245                 250                 255
   cca gtt gaa atc tgg cat cca gat cca gca aaa aat gag aac aca agg     816
   Pro Val Glu Ile Trp His Pro Asp Pro Ala Lys Asn Glu Asn Thr Arg
               260                 265                 270
   tac ttt ggc aat tac act gga ggc acg tgc acc cca ccc gtc ctg cag     864
   Tyr Phe Gly Asn Tyr Thr Gly Gly Thr Cys Thr Pro Pro Val Leu Gln
           275                 280                 285
   ttc aca aac acc ctg aca act gtg ctc cta gat gaa aat gga gtt ggg     912
   Phe Thr Asn Thr Leu Thr Thr Val Leu Leu Asp Glu Asn Gly Val Gly
       290                 295                 300
   ccc ctc agc aaa gga gag ggc cta tac ctc tcg agc gta gat ata atg     960
   Pro Leu Ser Lys Gly Glu Gly Leu Tyr Leu Ser Ser Val Asp Ile Met
   305                 310                 315                 320
   ggc tgg aga gtt aca aga aac tat gat gtc cat cac tgg aga ggg ctt     1008
   Gly Trp Arg Val Thr Arg Asn Tyr Asp Val His His Trp Arg Gly Leu
                   325                 330                 335
   ccc aga tat ttc aaa atc acc ctg aga aaa aga tgg gtc aaa aat ccc     1056
   Pro Arg Tyr Phe Lys Ile Thr Leu Arg Lys Arg Trp Val Lys Asn Pro
               340                 345                 350
   tat ccc atg gcc tcc ctc ata agt tcc ctt ttc aac aac atg ctc ccc     1104
   Tyr Pro Met Ala Ser Leu Ile Ser Ser Leu Phe Asn Asn Met Leu Pro
           355                 360                 365
   caa gtg cag ggc caa ccc atg gaa ggg gag aac acc cag gta gag gag     1152
   Gln Val Gln Gly Gln Pro Met Glu Gly Glu Asn Thr Gln Val Glu Glu
       370                 375                 380
   gtt aga gtg tat gat ggg act gaa cct gta ccg ggg gac cct gat atg     1200
   Val Arg Val Tyr Asp Gly Thr Glu Pro Val Pro Gly Asp Pro Asp Met
   385                 390                 395                 400
   acg cgc tat gtt gac cgc ttt gga aaa aca aag act gta ttt cct ccc     1248
   Thr Arg Tyr Val Asp Arg Phe Gly Lys Thr Lys Thr Val Phe Pro Pro
                   405                 410                 415
   ggg                                                                 1251
   Gly
<210> 10
   <211> 417
   <212> PRT
   <213> Künstliche Sequenz
   <223> Beschreibung der künstlichen Sequenz: WW-Domäne N-terminal an Polyomavirus VP1, das 4 der 6 Cysteine durch Serin ersetzt hat und den Austausch VP1-Thr 249 nach Cys hat (PyVP1-WW1)
<400> 10
   Met Ser Gly Trp Thr Glu His Lys Ser Pro Asp Gly Arg Thr Tyr Tyr
    1               5                  10                  15
   Tyr Asn Thr Glu Thr Lys Gln Ser Thr Trp Glu Lys Pro Asp Asp Gly
               20                  25                  30
   His Met Ala Pro Lys Arg Lys Ser Gly Val Ser Lys Ser Glu Thr Lys
           35                  40                  45
   Ser Thr Lys Ala Cys Pro Arg Pro Ala Pro Val Pro Lys Leu Leu Ile
       50                  55                  60
   Lys Gly Gly Met Glu Val Leu Asp Leu Val Thr Gly Pro Asp Ser Val
   65                  70                  75                  80
   Thr Glu Ile Glu Ala Phe Leu Asn Pro Arg Met Gly Gln Pro Pro Thr
                   85                  90                  95
   Pro Glu Ser Leu Thr Glu Gly Gly Gln Tyr Tyr Gly Trp Ser Arg Gly
               100                 105                 110
   Ile Asn Leu Ala Thr Ser Asp Thr Glu Asp Ser Pro Gly Asn Asn Thr
           115                 120                 125
   Leu Pro Thr Trp Ser Met Ala Lys Leu Gln Leu Pro Met Leu Asn Glu
       130                 135                 140
   Asp Leu Thr Cys Asp Thr Leu Gln Met Trp Glu Ala Val Ser Val Lys
   145                 150                 155                 160
   Thr Glu Val Val Gly Ser Gly Ser Leu Leu Asp Val His Gly Phe Asn
                   165                 170                 175
   Lys Pro Thr Asp Thr Val Asn Thr Lys Gly Ile Ser Thr Pro Val Glu
               180                 185                 190
   Gly Ser Gln Tyr His Val Phe Ala Val Gly Gly Glu Pro Leu Asp Leu
   195 200 205
   Gln Gly Leu Val Thr Asp Ala Arg Thr Lys Tyr Lys Glu Glu Gly Val
   210 215 220
   Val Thr Ile Lys Thr Ile Thr Lys Lys Asp Met Val Asn Lys Asp Gln
   225 230 235 240
   Val Leu Asn Pro Ile Ser Lys Ala Lys Leu Asp Lys Asp Gly Met Tyr
   245 250 255
   Pro Val Glu Ile Trp His Pro Asp Pro Ala Lys Asn Glu Asn Thr Arg
   260 265 270
   Tyr Phe Gly Asn Tyr Thr Gly Gly Thr Cys Thr Pro Pro Val Leu Gln
   275 280 285
   Phe Thr Asn Thr Leu Thr Thr Val Leu Leu Asp Glu Asn Gly Val Gly
   290 295 300
   Pro Leu Ser Lys Gly Glu Gly Leu Tyr Leu Ser Ser Val Asp Ile Met
   305 310 315 320
   Gly Trp Arg Val Thr Arg Asn Tyr Asp Val His His Trp Arg Gly Leu
   325 330 335
   Pro Arg Tyr Phe Lys Ile Thr Leu Arg Lys Arg Trp Val Lys Asn Pro
   340 345 350
   Tyr Pro Met Ala Ser Leu Ile Ser Ser Leu Phe Asn Asn Met Leu Pro
   355 360 365
   Gln Val Gln Gly Gln Pro Met Glu Gly Glu Asn Thr Gln Val Glu Glu
   370 375 380
   Val Arg Val Tyr Asp Gly Thr Glu Pro Val Pro Gly Asp Pro Asp Met
   385 390 395 400
   Thr Arg Tyr Val Asp Arg Phe Gly Lys Thr Lys Thr Val Phe Pro Pro
   405 410 415
   Gly